# EUROPEAN PATENT SPECIFICATION

(11) **EP 1 677 775 B1**
(45) Date of publication and mention of the grant of the patent: **07.12.2011**
(21) Application number: 04810055.6
(22) Date of filing: 27.10.2004
(51) Int. Cl.: A61K 31/095, A61K 31/198, A61P 17/00

(54) **THERAPEUTIC USE OF METHIONINE FOR THE TREATMENT OR PREVENTION OF MUCOSITIS**
THERAPEUTISCHE ANWENDUNG VON METHIONIN ZUR BEHANDLUNG ODER PRÄVENTION VON MUCOSITIS
UTILISATION THERAPEUTIQUE DE LA METHIONINE POUR LE TRAITEMENT OU LA PREVENTION DE LA MUCOSITE

(30) Priority: 27.10.2003 US 694436
(43) Date of publication of application: 12.07.2006
(73) Proprietor: THE BOARD OF TRUSTEES OF SOUTHERN ILLINOIS UNIVERSITY, Springfield, IL 62794-9230 (US)
(72) Inventor: CAMPBELL, Kathleen C. M., SIU School of Medicine, PO Box 19629, Springfield IL 62794-9629 (US)
(74) Representative: Smaggasgale, Gillian Helen
(86) International application number: PCT/US2004/035626
(87) International publication number: WO 2005/039554

(56) References cited:
- WO-A-96/01101
- WO-A-98/14182
- WO-A-02/060491
- WO-A-03/045334
- US-A- 4 961 926
- US-A- 5 667 791
- US-B1- 6 265 386
- GENTILE-RAMOS I: "[Treatment of "diaper erythema" with methionine]" ARCHIVOS DE PEDIATRIA DEL URUGUAY. AUG 1965, vol. 36, no. 8, August 1965 (1965-08), pages 519-528, XP008048587 ISSN: 0004-0584

## Description

### BACKGROUND OF THE INVENTION

The present invention relates to the use of protective agents in cancer chemotherapy in human and animal subjects. Protective agents are compounds that prevent, reduce, or otherwise ameliorate the toxic side effects associated with anti-cancer chemotherapy regimens in normal body cells while substantially preserving the anti-tumor properties of such therapies in vivo when administered prior to, concomitantly with, or subsequently to the commencement of such chemotherapeutic regimen. More specifically, the present invention relates to the use of D-methionine and structurally related compounds according to the claims as protective agents having protective, effect against skin damage, and mucositis-protective effects in conjunction with chemotherapy employing platinum-containing anti-neoplastic agents, such as cisplatin. The present invention also relates to the use of D-methionine and structurally related compounds according to the claims as protective agents having protective effects against radiation-induced hearing loss, as well as protective effects in ameliorating other radiation-induced side effects such as mucositis and skin damage.

### 1. Cisplatin Chemotherapy

Cisplatin (cis-diamminedichloroplatinum(II); CDDP) is a widely used antineoplastic agent. Cisplatin administration has increased both in the variety of cancer types for which it is employed and in the amount used in a given individual to achieve maximal therapeutic effect. See, Blumenreich et al., Cancer, vol. 55, pp. 1118-22 (1985); Forastiere et al., Cancer Chemo. Pharm., vol. 19, pp. 155-8 (1987); Gandara et al., Proc. Am. Assoc. Cancer Res. (959), Vol. 30, p. 241. (1989); Gandara et al., Anticancer Res., vol. 9, pp. 1121-8 (1989).

The toxic side effects of cisplatin have long been recognized and are widely reported. See, Lippman et al., "Clinical Trials of Cis-Diamminedichloroplatinum (NSC-119875)," Cancer Chemother. Rep., Part 1, vol. 57, pp. 191-200 (1973); and Hacker, The Toxicity of Anticancer Drugs, pp. 82-105, (Pergamon Press, 1991). These toxicities include a variety of peripheral neuropathies, myelosuppression, gastrointestinal toxicity, nephrotoxicity, and ototoxicity. See, Ozols and Young, Semin. Oncol., 12(4), Suppl. 6, pp. 21-30 (1985); Stewart et al., Am. J. Clin. Oncol., 10(6), pp. 517-19 (1987); Stoter et al., J. Clin. Oncol., 7(8), pp. 1099-1104 (1989). Initially, the primary dose-limiting factor was nephrotoxicity, but now the routine administration of mannitol, hypertonic saline, and high fluid administration have ameliorated, but not eliminated, that side effect. However, ototoxicity remains uncontrolled. See, Bajorin et al., J. Clin. Oncol., 5(10), pp. 1589-93 (1987); Fillastre et al., Toxicol. Lett., 46, pp. 163-75 (1989). Although nephrotoxicity can still be dose-limiting, currently the primary dose-limiting factor is ototoxicity. See, Blumenreich et al., Cancer, 55, pp. 1118-22 (1985); Forastiere et al., Cancer Chemo. Pharm., 19, pp. 155-8 (1987); Berry et al., J. Clin. Oncol., 8(9), pp. 1585-90 (1990).

The primary ototoxic effects of cisplatin appear to occur in the cochlea. Anatomical changes occur in both the stria vascularis and the organ of Corti. The primary histologic findings include hair cell degeneration and damage to the supporting cells that are dose-related. See, Anniko and Sobin, Am. J. Otol., 7, pp. 276-93 (1986). At high doses, total collapse of the membranous labyrinth can occur. See Id. In the organ of Corti, there is loss of outer and inner hair cells, with a propensity for outer hair cell loss in the basal turn, and alterations in the supporting cells and Reissner's membrane. See, Fleischman et al., Toxicol. Appl. Pharm., 33, pp. 320-32 (1975); Komune, "Potentiating Effects of Cisplatin and Ethacrynic Acid in Ototoxicity," Arch. Otolaryngol., 101, pp. 66-74 (1981); Estrem et al., Otolaryngol. Head Neck Surg., 89, pp. 638-745 (1981); and Schweitzer, Laryngoscope, 103, pp. 1-52 (1993). Estrem et al. also reported softening of the cuticular plate and an increased number of lysosomal bodies in the apical portion of the outer hair cell. However, the mechanisms inducing these changes are largely unknown.

For equivalent inner ear concentrations, cisplatin is the most ototoxic drug known. See, Moroso et al., J. otolaryngol., 12(6), pp. 365-9 (1983); Koegel, Am. J. Otol., 6(2), pp. 190-9 (1985); Anniko and Sobin, Am. J. Otol., vol. 7, pp. 276-93 (1986); and Griffin, Brit. J. Audio., 22, pp. 195-210 (1988). Generally, cisplatin ototoxicity is irreversible, its onset insidious, and the hearing loss may progress after discontinuation of the protocol. See, Schaefer et al., Cancer, 56(8), pp. 1934-39 (1985); Melamed et al., Cancer, 55, pp. 41-43 (1985); Pollera et al., Cancer Chemother. Pharmacol., 21, pp. 61-4 (1988); Aguilar-Markulis et al., J. Surg. Oncol., 16, pp. 111-23 (1981); and Moroso et al., J. Otolaryngol., 12(6), pp. 365-9 (1983). Hearing loss is usually permanent. See, Vermorken et al., Eur. J. Cancer Clin. Oncol., 19(1), pp. 53-58 (1983). Partial recovery may occur in some cases, but only one of 121 patients with hearing loss had complete recovery in a study by Aguilar-Markulis et al., supra. Hearing loss typically starts at the ultra high frequencies (9000 to 20000 Hz) and then progresses into the high conventional audiometric range, reducing the patient's ability to hear consonant but not vowel sounds. See, Fausti et al., Cancer, 53, pp. 224-31 (1984); Kopelman et al., Laryngoscope, 98, pp. 858-64 (1988); Laurell and Engström, Hearing Research, 38, 27-34 (1989); and Meyer, J. Clin. Oncol., 7(6), 754-760 (1989). An inability to understand speech and tinnitus are frequent complaints (Kopelman et al., supra). An increasing number of patients survive chemotherapy, but frequently with hearing impairment.

### 2. Nucleophilic Sulfur Protective Agents

Many sulfur-containing compounds (including substances with thio, thiol, and thioether groups) have been reported to provide CDDP nephroprotection in animal models. See, Anderson, et al., FASEB J., vol. 4, pp. 3251-5 (1990); Jones and Basinger, Anticancer Res., 9, pp. 1937-42 (1989); Jones et al., Cancer Chemo. Pharm., 17, pp. 38-42 (1986); Jones et al., Toxicology, 68, pp. 227-47 (1991); Jones et al., Anticancer Res., 11, pp. 449-54 (1991); Jones et al., Anticancer Res., 11, pp. 1939-42 (1991); and Jones et al., Fundam. Appl. Toxicol., 18, pp. 181-8 (1992). These compounds may act by preventing the CDDP-induced depletion of glutathione or the binding of CDDP to protein sulfhydryl groups. See, Hannemann et al., Toxicology, 51, pp. 119-32 (1988); Nakano et al., Jpn. J. Pharmacol., 50, pp. 87-92 (1989); Gandara et al., Anticancer Res., 9, pp. 1121-8 (1989); Ravi et al., Pharmacologist, 33(3), p. 217 (1991); and Schweitzer, Laryngoscope, 103, pp. 1-52 (1993).

Additionally, sodium thiosulfate (STS) and diethyldithiocarbamate (DDTC) provide good CDDP otoprotection in animals. See, Otto et al., Hearing Research, 35, pp. 79-86 (1988); Church et al, Hearing Research 86(1,2), pp. 195-203 (1995); and Rybak et al., Fundam. Appl. Toxicol., 26, pp. 293-300 (1995). Unfortunately, STS may reduce CDDP tumoricidal action and may exacerbate CDDP-induced weight loss and mortality. See, Pfeifle et al., J. Clin. Oncol., 3, pp. 237-44 (1985); Aamdal et al., Cancer Treat., Rev. 14, pp. 389-95 (1987); and Otto et al., supra. DDTC does not interfere with antitumor action, but can produce severe side effects. See, Dedon et al., "Diethyldithiocarbamate (DDTC) Reversal of Cisplatin (DDP) Nechrotoxicity," AACR Abstracts, 1470, p. 371 (1985); Borch et al., Organ Directed Toxicities of Anticancer Drugs, 3d ed., pp. 190-20 (Matinus Nijhoff Publishing,; 1988); Rothenberg et al., J. Nat'l. Cancer Inst., 80, pp. 1488-92 (1988); Qazi et al., J. Nat'l. Cancer Inst., 80(18), pp. 1486-92 (1988); and Berry et al., Proceedings of ASCO, (266) 8, 69 (1989).

### D-Methionine

D-methionine is a sulfur-containing nucleophile that provides highly effective CDDP nephroprotection in animals without decreasing anti-tumor action. See, Jones and Basinger, Anticancer Res., 9, pp. 1937-42 (1989). D-methionine was also the most effective CDDP nephroprotectant that did not interfere with CDDP tumoricidal action out of nearly 40 sulfur-containing agents tested in a series of studies by Jones and colleagues. See, Jones et al., Cancer Chemo. Pharm., 17, pp. 38-42 (1986) Jones and Basinger, Anticancer Res. , 9, pp. 1937-42 (1989); Jones et al., Toxicology, 68, pp. 227-47 (1991); Jones et al., Anticancer Res., 11, pp. 449-54 (1991); Jones et al., Anticancer Res., 11, pp. 1939-42 (1991); and Jones et al., Fundam. Appl. Toxicol., 18, pp. 181-8 (1992).

### Sulfur-Containing Protective Agents and the Modulation of Cisplatin-Induced Toxicity

Studies indicate that individual sulfur-containing protective agents may only be effective in reducing specific types of toxicity, such as nephrotoxicity, while remaining ineffective in blocking other platinum-related complications such as peripheral neuropathy and ototoxicity. In addition, an agent which is effective as a regional chemoprotector following site-specific (intraperitoneal) usage of platinum-containing compounds such as CDDP may fail to provide adequate systemic protection, or may inhibit antitumor activity. See, Schweitzer, Laryngoscope, 103, pp. 1-52 (1993).

Not all sulfur-containing compounds provide protection against all of CDDP's toxicities, and it is not possible to predict which protective agents will be effective or ineffective for this purpose. For example, cefoxitin does not provide nephroprotection. See, Jones et al., Fundam. Appl. Toxicol., 18, pp. 181-8 (1992). Ethyl-L-cysteinate and N-(2-mercapto-propionyl)glycine exacerbate CDDP nephrotoxicity. See, Jones and Basinger, Anticancer Res., 9, pp. 1937-42 (1989). 2-(methylthio)nicotinic acid does not provide nephroprotection in rats. See, Jones et al., Anticancer Res., 11, pp. 449-54 (1991). The sodium salt of penicillin G does not protect against CDDP nephrotoxicity or weight loss. See, Jones et al., Fundam. Appl. Toxicol., 18, pp. 181-8 (1992). Similarly, thiamine-HCl does not protect against cisplatin nephrotoxicity or weight loss. See Id.

Furthermore, sulfur-containing compounds protective against one type of CDDP toxicity frequently do not protect against other CDDP toxicities, and it is not possible to predict the specific antitoxic effectiveness of such compounds. Cephalexin protects against CDDP-induced kidney dysfunction and weight loss, but curiously does not prevent kidney pathology. See, Jones et al., Fundam. Appl, Toxicol., 18, pp. 181-8 (1992). Cefoxitin provides some protection against CDDP-induced weight loss, but does not protect against CDDP nephrotoxicity. See Id. The sodium salt of penicillin G does not protect against either CDDP-induced nephrotoxicity or weight loss. Id. Sulfathiazole provides protection against CDDP nephrotoxicity, but not weight loss. Id.

WR2721 provides excellent CDDP nephroprotection, but does not ameliorate nausea and vomiting. See, Mollman et al., Cancer 61, pp. 2192-5 (1988) and Glover et al., J. Clin. Oncol., 5, pp. 574-8 (1987). Nor does WR2721 seem to provide CDDP otoprotection. Glover et al. found mild to severe hearing loss in 20 of 36 patients receiving WR2721 prior to CDDP although nephroprotection was obtained. Rubin et al., J. Laryngol. Otol., 109(8), pp. 744-47 (1995), reported a 45% incidence of significant hearing threshold shift in patients pretreated with WR2721 prior to CDDP administration. Unfortunately, neither the Glover et al. nor Rubin et al. studies employed a control group, and both reported a high incidence of ototoxicity in patients receiving WR2721. In hamsters, Church et al, Hearing Research 86(1,2), pp. 195-203 (1995), reported no WR2721 protection from ototoxicity or mortality.

Even when a sulfur-containing agent is found to be protective, its side effects can be so severe that clinical applicability is precluded. In addition, even among agents that provide CDDP otoprotection, the protection may be so inconsistent and/or the side effects so great that they would not be used clinically. For example, DDTC provides protection against CDDP-induced nephrotoxicity and ototoxicity, but the protection against ototoxicity may only be partial and its side effects are severe. See, Qazi et al., J. Nat'l. Cancer Inst., 80(18), pp. 1486-92 (1988); Berry et al., Proceedings of ASCO, (266) 8, 69 (1989); Gandara et al., Proc. Am. Assoc. Cancer Res. (959), Vol. 30, p. 241 (1989); Gandara et al., Anticancer Res., 9, pp. 1121-8 (1989); Gandara et al., Sem. Oncol., 18(1), pp. 49-55 (1991); Church et al, Hearing Research 86(1,2), pp. 195-203 (1995); Ravi et al., Otolaryngol. Head Neck Surg., 107(2), p. 232 (1992); and Rothenberg et al., J. Nat'l. Cancer Inst., 80, pp. 1488-92 (1988). If DDTC dosing is reduced to ameliorate its side effects, adequate protection from CDDP side effects may not occur. See, Paredes et al., J. Clin. Oncol., 6, p. 955 (1988). Similarly, disulfiram (Antabuse), which can be used as a precursor for its metabolite DDTC, can cause sensorimotor neuropathy and reversible confusion that can be dose-limiting. See, Argov et al., New. Engl. J. Med., 301(8), pp. 409-13 (1979); and Stewart et al., Am. J. Clin. Oncol., 10(6), pp. 517-19 (1987). Consequently, it is unlikely that DDTC will be widely used clinically as a CDDP chemoprotectant. In contrast, as described below, D-methionine provides complete otoprotection without apparent adverse side effects.

Finally, many sulfur-containing compounds inhibit the anti-tumor action of CDDP, and it is not possible to predict which agents will or will not act in this manner. Thus, many agents that provide CDDP protection are not clinically useful. For example, Captropril protects against CDDP nephrotoxicity, but reacts immediately with CDDP to form a precipitate if coadministered, thereby precluding anti-tumor efficacy. See, Jones et al., Fundam. Appl. Toxicol., 18, pp. 181-8 (1992). L-methioninamide provides excellent CDDP nephroprotection, but impairs CDDP anti-tumor action. See, Jones et al., Anticancer Res., 11, pp. 449-54 (1991). Metallothionein, a sulfur-containing compound the synthesis of which is induced by administration of bismuth subnitrate, provides CDDP nephroprotection, but also inhibits CDDP anti-tumor action. See, Naganuma et al., Cancer Res., 47, pp. 983-7 (1987); Boogaard et al., Biochem. Pharm., 41(3), pp. 369-75 (1991); Satoh et al., Cancer Res., 53, pp. 1829-32 (1993); and Endresen et al., Acta Pharmacol. Toxicol., 55(3), pp. 183-87 (1984). STS reduces CDDP nephrotoxicity and ototoxicity, although some authors report inadequate otoprotection. See, Pfeifle et al., J. Clin. Oncol., 3, pp. 237-44 (1985); Howell et al., Ann. Int. Med., 97(6), pp. 845-51 (1982); Otto et al., Hearing Research, 35, pp. 79-86 (1988); Church et al, Hearing Research 86(1,2), pp. 195-203 (1995); and Markman et al., Cancer, 56, pp. 2364-8 (1985). However, STS will probably not be clinically useful as coadministration with CDDP reduces the latter's tumoricidal action, and two route administration does not provide nephroprotection. See, Pfeifle et al., J. Clin. Oncol., 3, pp. 237-44 (1985); Aamdal et al., Cancer Treat., Rev. 14, pp. 389-95 (1987); and Jones et al., Anticancer Res., 11, pp. 449-54 (1991). Even in the absence of other agents, STS may also increase mortality and induce weight loss. See, Otto et al., Hearing Research, 35, pp. 79-86 (1988). Biotin, another sulfur-containing compound that provides good CDDP nephroprotection, inhibits anti-tumor activity. See, Jones et al., Fundam. Apρl. Toxicol., 18, pp. 181-8 (1992).

Thus, a variety of sulfur-containing compounds can act as protective agents for particular toxicities. A comparison of C-SH- and C-S-C-containing compounds demonstrated that the C-S-C- group was more effective in preventing nephrotoxicity in rats. See, Jones and Basinger, Anticancer Res., 9, pp. 1937-42 (1989). However, not all of the compounds possessing the C-S-C- group were found to be effective cisplatin antagonists.

The foregoing discussion demonstrates that it is not possible to predict reliably which particular sulfur- , containing nucleophile will exhibit a platinum-containing compound protective effect in any particular type of cell, tissue, or organ. Indeed, individual compounds seem to exert their protective effects only in certain tissues. Thus, the ability of any particular nucleophilic sulfur compound to act as a protective agent in any particular tissue can only be determined by direct experimentation. Of course, such compound will only be of value if it does not substantially reduce the anti-tumor efficacy of cisplatin or related anti-tumor platinum-containing compounds.

Deegan et al., Toxicology, 89, pp. 1-14 (1994), demonstrated that male Wistar rats receiving a single intraperitoneal dose of cisplatin-methionine at a 1:5 ratio by weight did not exhibit cisplatin-induced nephrotoxicity. Their results indicated that cisplatin-methionine is significantly cytotoxic, yet lacks cisplatin-associated renal toxicity. These workers suggested a role for either methionine co-treatment or cisplatin-methionine compounds in the treatment of human cancers. However, they neither disclosed nor suggested the specific otoprotective, weight loss-protective, gastrointestinal-protective, neuroprotective, alopecia-protective, or survival-enhancing effects of D-methionine surprisingly discovered by the present inventor. Nor did they provide any motivation to investigate D-methionine as an otoprotectant, weight loss-protectant, survival-enhancing agent, etc., or any reasonable expectation that methionine could act in these manners during cisplatin administration. Finally, Deegan et al. provided no guidance or suggestion as to how methionine could be used as a protective agent for various toxicities in humans, as described herein. As noted by Schweitzer, Laryngoscope, 103, pp. 1-52 (1993) at page 12, while various nucleophilic sulfur protective agents have been shown to be effective in blocking or reversing the renal toxicity of CDDP while retaining the chemotherapeutic activity of the drug, each agent has to be considered individually. The effects on antineoplastic activity, individual CDDP toxicities, and appropriate dosing schedules need to be determined on a per se basis for each compound.

In view of the foregoing, the utility of D-methionine as a highly effective otoprotectant, weight lose protectant, gastrointestinal protectant, neuroprotectant, alopecia protectant, and survival-enhancing agent which does not interfere with anti-tumor activity, and which does not appear to cause any serious side effects, could not have been predicted. In fact, the discovery of D-methionine's beneficial effects is surprising in view of the many significant problems, discussed above, encountered with previously described sulfur-containing nucleophiles that preclude their clinical use.

### SUMMARY OF THE INVENTION

The present inventor has addressed the long-felt need in the art for protective agents effective in preventing or ameliorating various toxic effects of cisplatin and other platinum-containing anti-tumor compounds, but which do not significantly affect the antineoplastic activity of these compounds, and which do not themselves cause deleterious side effects as a result of their administration. She has also addressed the long-felt need in the art for protective agents effective in preventing or ameliorating various toxic effects of radiation. She has surprisingly discovered that D-methionine, and structurally related compounds, can be used as an otoprotectant, a weight loss protectant, a gastrointestinal protectant, a neuroprotectant, an alopecia protectant, a mucositis protectant and a survival-enhancing agent during or after treatment of a mammal with such compounds, or during or after exposure of a mammal to radiation.

Accordingly, in one aspect, the present invention relates to use of a protective agent comprising methionine or a methionine-like moiety to prepare a pharmaceutical composition for the prevention or reduction of mucositis in a human or animal patient wherein the methionine-like moiety has the structural formula: 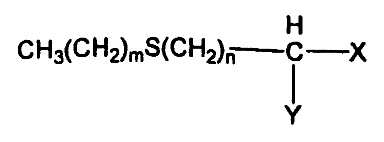 wherein m is an integer from 0 to 3; n is an integer from 1 to 3; X = -OR¹, -OCOR¹, - COOR¹, -CHO, -CH(OR¹)₂, or -CH₂OH Y = -NR²R³; R¹ = H or a substituted or unsubstituted, straight or branched chain alkyl group having 1 to 6 carbon atoms; R² = H or a substituted or unsubstituted, straight or branched chain acyl group having 1 to 6 carbon atoms; and R³ = H or a substituted or unsubstituted, straight or branched chain acyl group having 1 to 6 carbon atoms; or a pharmaceutically acceptable salt thereof.

In another embodiment, the present invention is directed to use of a protective agent comprising methionine or a methionine-like moiety to prepare a pharmaceutical composition for the prevention or reduction of skin damage in a human or animal patient wherein said human or animal patient is over exposed to radiation and wherein the methionine-like moiety has the structural formula: 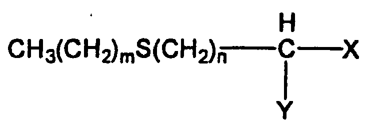 wherein m is an integer from 0 to 3; n is an integer from 1 to 3; X = -OR¹, -OCOR¹, - COOR¹, -CHO, -CH(OR¹)₂, or -CH₂OH Y = -NR²R³; R¹ = H or a substituted or unsubstituted, straight or branched chain alkyl group having 1 to 6 carbon atoms; R² = H or a substituted or unsubstituted, straight or branched chain acyl group having 1 to 6 carbon atoms; and R³ = H or a substituted or unsubstituted, straight or branched chain acyl group having 1 to 6 carbon atoms; or a pharmaceutically acceptable salt thereof wherein the protective agent is administered orally to said patient.

In other embodiments, the protective agent described above is selected from the group consisting of L-methionine, a mixture of D-methionine and L-methionine, normethionine, homomethionine, methioninol, ethionine, a pharmaceutically acceptable salt thereof, and a combination thereof.

The above and other objects, features, and advantages of the present invention will be better understood from the following detailed description taken in conjunction with the accompanying drawings.

### BRIEF DESCRIPTION OF THE DRAWINGS

FIGS. 1A-1E show ABR post-test thresholds (means t 1 S.D.) developed in Example 1 for the various animal groups for all stimuli including: 1A) clicks; 1B) 1000 Hz tonebursts; 1C) 4000 Hz tonebursts; 1D) 8000 Hz tonebursts; and 1E) 14000 Hz tonebursts. * indicates significantly different from the CDDP-treated controls at the p≤01 level.
FIGS. 2A-2F are SEM photomicrographs depicting results for Example 1 of: 2A) middle turn of untreated control; 2B) middle turn of treated control (16 mg/kg CDDP); 2C) middle turn of animal administered 300 mg/kg D-Met prior to the 16 mg/kg CDDP dose; 2D) basal turn of untreated control; 2E) basal turn of treated control (16 mg/kg CDDP); and 2F) basal turn of animal administered 300 mg/kg D-Met prior to the 16 mg/kg CDDP dose.
FIG. 3 shows the average weight loss in grams for the various animal groups studied in Example 1. indicate significantly different from the CDDP-treated controls at the p≤ . 01 level.
FIGS. 4A and 4B show the results of Example 2 for cell growth rates and viability of irradiated and control cells in the presence or absence of D-methionine.
FIG. 5 is a graph illustrating the percentage of cells in Example 3 having an apoptotic phenotype.
FIG. 6 shows results of the evaluation and quantification of lip erythema resulting from pre-treatment and post-treatment of the animals of Example 4 with D-methionine.

### DETAILED DESCRIPTION OF THE INVENTION

As described herein, Applicant has demonstrated that D-methionine prevents CDDP-induced ototoxicity, reduces CDDP-induced weight loss, protects against CDDP-induced gastrointestinal toxicity, mucositis, neurotoxicity, and alopecia, and improves survival during CDDP treatment in a mammal. Applicant has further demonstrated that D-methionine will be effective in the treatment of radiation-induced ototoxicity, as well as in ameliorating other radiation-induced side effects such as neural damage, alopecia, gastrointestinal disorders, mucositis and in improving patient survival.

As used herein, the term "ototoxicity" includes, but any detrimental or pathologic change in the structure or function of the ear, including changes in hearing and balance. Auditory functional changes can include, hearing loss or other changes in auditory threshold for any stimulus, perception of sound including recruitment (abnormal growth in.the perception of loudness), ability to identify, localize, recognize, distinguish between, or process sounds, and/or distortion of sounds or any abnormality as measured by conventional auditory tests. This term also includes tinnitus (ringing or noises in the ear), which includes any perception of sound that is not in response to an external signal. Further, ototoxicity includes any perceived or measured functional change in the balance or vestibular system, including, but not limited to, either induced or spontaneous vertigo, dysequilibrium, increased susceptibility to motion sickness, nausea, vomiting, nystagmus, syncope, lightheadedness, dizziness, difficulty in visual tracking secondary to vestibular or balance disorder or abnormality as measured on any test of vestibular or balance function. Structural changes can include any intra- or extra-cellular, multicellular, or organ change in the auditory or vestibular pathways from the external ear up through and including the cortex and all pathways in between.

The term "otoprotective agent" refers to an agent that prevents, ameliorates, or otherwise/protects against ototoxicity.

The term "neurotoxicity" includes, any detrimental or pathologic change in the structure or function in the neurologic system or any part thereof. Neurologic functional changes can include, neuropathy, either central or distal, including a common "stocking and glove" pattern, tingling, loss of sensation, numbness, decreased vibratory sensation, decreased deep tendon reflexes, sensory ataxia, neuritis, focal encephalopathy, aphasia, autonomic neuropathy, postural hypotension, a myasthenia-like syndrome, muscle cramps, headache, seizures, blindness or visual disturbance secondary to disorder of the optic or visual neurological pathway, papilledema, hearing loss secondary to disorder of the auditory neurologic pathway, and/or loss of the sensation of taste. Structural changes can include intra- or extra-cellular, multicellular, or organ changes, anywhere in the neurologic system, including both peripheral and central systems. Neurotoxicity can manifest itself during or after the course of treatment with platinum-containing anti-tumor compounds.

The term "neuroprotective agent" refers to an agent that prevents, ameliorates, or otherwise protects against neurotoxicity.

The term "gastrointestinal toxicity" includes, any detrimental or pathologic change in the structure or function in the gastrointestinal system or any part thereof. Gastrointestinal changes include, for example, current or delayed nausea, vomiting, esophageal reflux, stomatitis, bleeding along the gastrointestinal tract, diarrhea, weight loss, and/or anorexia. Gastrointestinal toxicity can manifest itself during or after the course of treatment with platinum-containing anti-tumor compounds.

The term "gastrointestinal-protective agent" refers to an agent that prevents, ameliorates, or otherwise protects against gastrointestinal toxicity.

The term "mucositis" refers to swelling, irritation or ulceration of the mucosal cells in the body. Generally, mucositis can occur in the middle ear, eyes, nose, sinuses, vagina, urinary tract and anywhere along the digestive tract from the mouth to the anus. More particularly mucositis can occur due to tissue irritation or swelling caused by an appliance (e.g., oral mucositis from dentures and mucositis due to catheters) As used herein, the term mucositis generally encompasses all forms of mucositis including oral mucositis (i.e., swelling, irritation or ulceration of oral mucosa), esophageal mucositis (i.e., swelling, irritation or ulceration of esophageal mucosa), gastrointestinal mucositis (i.e., swelling, irritation or ulceration of gastrointestinal mucosa) and aural mucositis (i.e., swelling, irritation or ulceration of the middle ear or round window membrane of the ear), mucositis of the eyes, mucositis of the nasal cavity and sinuses, and mucositis of the vaginal and urinary tracts.

The term "mucositis-protective agent" refers to an agent that prevents, ameliorates, or otherwise protects against mucositis (e.g., oral mucositis, esophageal mucositis and/or gastrointestinal mucositis).

### Methionine and Its Derivatives

D-methionine has been administered to humans for various purposes. For example, C-labeled D-methionine has been used for radiographic imaging, and DL-methionine has been administered for parenteral nutrition. See, Meyer et al., Eur. J. Nucl. Med., 10, 373-6 (1985); and Printen et al., Am. J. Clin. Nutr., 32, pp. 1200-05 (1979). D-methionine has also been safely administered to humans orally for nutritional studies. See, Kaji et al., Res. Comm. Chem. Path. Pharm., 36(1), pp. 101-9 (1987); Kies et al., J. Nutr., 105, pp. 809-14 (1975); and Stegink et al., J. Nutr., 116, pp. 1185-92 (1986). Oral methionine is sold as an over the counter preparation to control urinary pH. See, Drug Facts and Comparisons, 3d ed., p. 2115 (J.P. Lippincott Company, St. Louis, 1991). The contraindications-are-for patients with a history of liver disease, and that high dosage methionine may inhibit growth in children when given for an extended time period.

The compounds structurally related to D-methionine that can be employed in the present invention are those containing the C-S-C- (thioether) moiety having the structural formula: 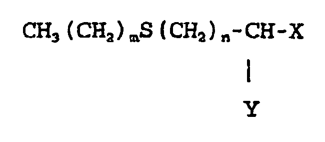 wherein m is an integer from 0 to 3; n is an integer from 1 to 3; X = -OR¹, -OCOR¹, -COOR¹, -CHO, -CH(OR¹)₂, or -CH₂OH, Y = -NR²R³; R¹ = H or a substituted or unsubstituted, straight or branched chain acyl group having 1 to 6 carbon atoms, preferably 1 to 4 carbon atoms; R² = H or a substituted or unsubstituted, straight or branched chain acyl group having 1 to 6 carbon atoms, preferably 1 to 4 carbon atoms; and R³ = H or a substituted or unsubstituted, straight or branched chain acyl group having 1 to 6 carbon atoms, preferably 1 to 4 carbon atoms; or a pharmaceutically acceptable salt thereof.

The acyl groups described herein, either alone or containing the various substituents defined herein, can contain from one to six carbon atoms in the principal chain, and up to about 15 carbon atoms total. The lower alkyl groups include, for example, methyl, ethyl, propyl, isopropyl, butyl, hexyl, cyclopropyl, cyclopentyl, cyclohexyl, and the like. Substituents of the substituted acyl groups described herein can include, for example, groups selected from alkyl, cycloalkyl, alkenyl, alkynyl, aryl, heteroaryl, O, S, N, P, or halogen (Cl, F, Br, or I) atoms. Optionally, these substituent alkyl, cycloalkyl, etc., groups can be substituted with O, S, N, P, or halogen (Cl, F, Br, or I) atoms. These substituent alkyl, cycloalkyl, groups include, for example, lower alkoxy groups such as methoxy, ethoxy, and butoxy, and groups such as halo, nitro, amino, and keto.

The alkenyl groups described herein, either alone or with the various substituents defined herein, are preferably lower alkenyl containing from two to six carbon atoms in the principal chain, and up to about 15 carbon atoms total They can be substituted, straight, or branched chain, and include ethenyl, propenyl, isopropenyl, butenyl, isobutenyl, hexenyl.

The alkynyl groups described herein, either alone or with the various substituents defined herein, are preferably lower alkynyl containing from two to six carbon atoms in the principal chain, and up to about 15 carbon atoms total. They can be substituted, straight or branched chain, and include ethynyl, propynyl, butynyl, isobutynyl, hexynyl.

The aryl moieties described herein, either alone or with various substituents defined herein, can contain from about 6 to about 15 carbon atoms, and include phenyl. Substituents include alkanoxy, protected hydroxy, halogen, alkyl, aryl, alkenyl, acyl, acyloxy, nitro, amino, amido, etc. Phenyl is a preferred aryl.

The heteroaryl moieties described herein, either alone or with various substituents defined herein, can contain from about 5 to about 15 atoms, and include, furyl, thienyl, pyridyl. Substituents include alkanoxy, protected hydroxy, halogen, alkyl, aryl, alkenyl, acyl, acyloxy, nitro, amino, and amido.

The acyloxy groups described herein can contain alkyl, cycloalkyl, alkenyl, alkynyl, aryl, or heteroaryl groups.

The carbon atoms, i.e., the methyl and methylene groups, constituting the principal backbone of the methionine or methionine-like moiety can also be substituted as variously described above.

Examples of such methionine protective agents include , L-methionine, a mixture of D-methionine and L-methionine, normethionine, homomethionine, methioninol, , ethionine, or pharmaceutically acceptable salts thereof. Methionine protective agents of the present invention can be in the D-, L-, or DL- form. D-methionine is a preferred compound.

Collectively, methionine, along with the other compounds discussed above, can be referred to as "methionine protective agents." These compounds can be used alone or in various combinations with one another in the methods described herein.

In another embodiment, said protective agents are selected from the group consisting of N-acetylcysteine (NAC), acetyl-L-carnitine (ALCAR), lipoic acid or combinations thereof. In combination with the methionine protective agents described above.

These compounds can be administered in combination with the other drug compounds discussed herein, in the form of the water-soluble acid, free base, or as physiologically acceptable salts, including acid addition salts formed with organic and inorganic acids, for example, hydrochlorides, hydrobromides, sulfates, phosphates, citrates, fumarates, and maleates, and cations such as sodium, potassium. These compounds can be formulated for administration to humans and animals with pharmaceutically acceptable carriers, excipients, and diluents, such as sterile distilled water, Ringer's solution, normal saline, 5% glucose, dextrose, fructose, sucrose, etc., and mixtures thereof, as is well known in the art. Antimicrobial agents, preservatives, etc., can also be included. Compositions for oral administration can include coloring and flavoring agents. Additional methods of formulating compounds of the present invention for administration in the methods described herein can be found, for example, in Remington's Pharmaceutical Sciences, Fifteenth Edition, Mack Publishing Company, Easton, Pennsylvania, 1975.

### Anti-tumor Platinum Compounds

Cisplatin (CDDP; *cis-*diamminedichloro-platinum(II)) is currently the anti-tumor platinum coordination compound most frequently employed in the therapy of testicular cancer, ovarian tumors, and a variety of other cancers. Methods of employing CDDP clinically are well known in the art. See, Nicolini, M. (Ed.) "Platinum and Other Metal Coordination Compounds in Cancer Chemotherapy. Proceedings of the 5th International Syumposium on Platinum and Other Metal-Coordination Compounds in Cancer Chemotherapy, Padua, Italy, June 29-July 2, 1987," (Martincis Nijhoff Publishing, Boston 1987). For example, CDDP can be administered in a single day over a six hour period, once per month, by slow intravenous infusion. For localized lesions, CDDP can be administered by local injection. Intraperitoneal infusion can also be employed. CDDP can be administered in doses as low as 10 mg/m² per treatment if part of a multi-drug regimen, or if the patient has an adverse reaction to higher dosing. At the low end, a more common clinical dose is about 30 mg/m²; the high end of the range is about 120 to about 150 mg/m² per treatment. When used in conjunction with D-methionine or other methionine protective agents, these dosages can be increased.

CDDP is representative of a broad class of water-soluble, platinum coordination compounds well known in the art that provide platinum in the form of an ion having anti-tumor activity. Among the anti-tumor platinum coordination compounds described in the literature which are useful in the methods of the present invention are, for example, *trans*-diaminedichloro-platinum(II), *cis*-diamine-diaquaplatinum(II)-ion, cis-diaminedichloroplatinum(II)-ion, chloro(diethylenetriamine)-platinum(II) chloride, dichloro(ethylenediamine)-platinum(II), diammine(1,1-cyclobutanedicarboxylato)-platinum(II) (carboplatin), spiroplatin, dichlorotrans-dihydroxybisisopropolamine platinum IV (iproplatin), diammine(2-ethylmalonato)-platinum(II), ethylenediamine-malonatoplatinum(II), aqua(1,2-diaminodiclohexane)-sulfatoplatinum(II), (1,2-diaminocyclohexane)malonato-platinum(II), (4-carboxyphthalato)(1,2-diaminocyclo-hexane)-platinum(II), (1,2-diaminocyclohexane)-(isocitrato)platinum(II), (1,2-diaminocyclohexane)-cis(pyruvato)platinum(II), and (1,2-diaminocyclohexane)-oxalatoplatinum(II).

### Antineoplastic Agents

Various antineoplastic agents and combinations thereof are used as chemotherapeutic treatments. These agents can be used in combination with other chemotherapeutic treatments or in combination with radiation therapy. When used alone and/or in combination with other therapies, agents other than platinum-containing agents can cause mucositis and other side effects. Thus, the methods of the prevention are useful to treat mucositis induced by the administration of L-asparaginase, Ara-C, busulfan, cyclophosphamide, docetaxel, doxorubicin, edatrexate, etoposide, fludarabine, fluorouracil, gencitabine, idarubicin, ifosamide, irinotecan, leucovorin, melphalan, methotrexate, mitomycin C, mitoxantrone, oxaliplatin, paclitaxel, raltitrexed, thiotepa, vinorelbine. (Cancer, 2004, vol. 100(9), pp. 2007-2008)

### Radiation

Generally, overexposure to electromagnetic radiation from a variety of sources can cause mucositis, ototoxicity, skin damage and other tissue damage. Various types of radiation exposure are radiation therapy, ultraviolet (UV) radiation, microwave radiation, gamma radiation, X-rays.

Exposure to radiation, whether intentional, as in radiation therapy, or unintentional, as by accident, war, or terrorist act can result in ototoxicity, as well as neural damage (neurotoxicity), alopecia, gastrointestinal disorders, mucositis, skin damage and reduced patient survival. Although physical rather than chemical, radiation can be considered another "ototoxin" in view of its toxicity to the ear and hearing. Radiation-induced hearing loss is more likely to involve the middle ear than is hearing loss caused by platinum-containing compounds or loop diuretics; however, cochlear and neural problems can also occur.

Radiation-induced ototoxicity can occur as a result of exposure to 35-40 Gy of radiation or higher, either as a single or cumulative dose. Radiation-induced gastrointestinal toxicity, which is similar to that occurring during chemotherapy, includes electrolyte loss, secondary infections, bloody diarrhea, and gastrointestinal bleeding, and can occur upon exposure to a radiation dose of from about 5 Gy to about 20 Gy, or higher.

### Mucositis

Unintentional radiation exposures such as those occurring by accident, war, terrorist act and even prolonged exposure to the sun; and particularly intentional radiation exposures such as radiation doses delivered during chemotherapy and radiation therapy designed to kill cancer cells, induce unavoidable changes in the surrounding normal tissues, which can compromise overall cell function and host defenses thereby leading to severe complications. For example, chemotherapy and radiation therapy at conventional levels or at higher-dosed levels used in conditioning regimens (e.g., total body radiation in preparation for bone marrow transplantation [BMT]), often results in erythema, atrophy, and ulceration of the mucosa of the digestive tract, a condition generally referred to mucositis. Mucositis may manifest itself anywhere in the digestive tract between the mouth and the anus, for example, in oral mucosa, esophageal mucosa or in gastrointestinal mucosa. Although the description below will disclose with particularity the use of methionine protective agents for treating or preventing oral mucositis (i.e., erythema, atrophy or ulceration of oral mucosa)., it should be recognized that the principles described herein are generally applicable to other forms of mucositis.

Approximately one half of all patients who receive chemotherapy and/or radiation therapy develop such severe oral mucositis that it becomes dose-limiting. Thus, durable disease remission and cure rates may be enhanced if more intensive therapies could be used without the untoward consequences of dose-limiting oral mucositis.

Without being held to a particular theory, it is believed that the pathophysiology of oral mucositis results from a complex interaction of local tissue damage, the local oral environment, the patient's level of myelosuppression, and the patient's intrinsic predisposition to develop the condition. One biological model for oral mucositis is based on 4 interrelated phases, including an initial inflammatory/vascular phase, an epithelial phase, an ulcerative/bacteriological phase, and a healing phase. In the inflammatory phase, the chemotherapeutic agents lead to the release of interleukin 1 (IL-1) and tumor necrosis factor-alpha (TNF-alpha) from the epithelium. IL-1 mediates inflammation and dilates vessels, potentially increasing the concentration of chemotherapeutic agents at the site. TNF-alpha causes tissue damage, perhaps in an escalating fashion. Other cytokines that are putatively important in the pathogenesis of oral mucositis and that may have potential therapeutic application include interleukin 11 (IL-11) and transforming growth factor-beta3 (TGF-beta3).

During the epithelial phase, chemotherapy and/or radiation exposure retard cell division in the oral mucosal epithelium, resulting in reduced epithelial turnover and renewal. The result is erythema from increased vascularity and epithelial atrophy 4-5 days after the initiation of chemotherapy. Microtrauma from day-to-day activities such as speech, swallowing, and mastication leads to ulceration. During the ensuing ulcerative/bacteriological phase (during which time neutropenia has developed), putative bacterial colonization of ulcerations occurs, resulting in the flow of endotoxins into mucosal tissues and the subsequent release of more IL-1 and TNF-alpha. During the fourth and final healing phase, cell proliferation occurs with re-epithelialization of ulcers, reconstitution of the white cells effects local control of bacteria, and the ulcers resolve.

Furthermore, mucositis can occur secondary to trauma or other wounds, irritating appliances (e.g., dentures) or other causes of epithelial damage and irritation or inflammation of the mouth or digestive tract. Additionally, mucositis can occur secondary to impaired immune function, low salivary production, periodontal disease or dental caries. "Poor gut function" can also cause gastrointestinal mucositis in addition to the above items.

### Skin Damage

In addition to the side effects described above, treatment of a patient with chemotherapeutic agents and/or radiation exposure can cause skin damage in a patient. The radiation exposure can be accidental or intentional. It can arise in the course of war, terrorist attack, prolonged exposure to the sun, or by exposure to radiation doses delivered during chemotherapy and radiation therapy designed to kill cancer cells. A range of skin problems can result. In particular, radiation-exposure can cause mild to severe erythema (i.e., redness of the skin due to capillary dilation), dry desquamation (e.g., burns, dry scaly patches), wet desquamation (e.g., blisters, wet scaly patches) and swelling of the affected or exposed area. Sunburn may be considered a form of erythema.

### Administration of Methionine Protective Agents

The methionine protective agents of the present invention can generally be administered by any of a wide variety of means. For example, it is contemplated by the present invention that the methionine protective agents may be provided to a patient by oral administration, parenteral administration, bucchal administration, sublingual administration, rectal administration, topical administration, nasal administration, via'an eye drop, nose drop or by inhalation. In a preferred embodiment, the protective agent is administered orally or parenterally, for example intraperitoneally, by intravenous injection, intravenous infusion, etc., as described in Remington's Pharmaceutical Sciences, Fifteenth Edition, Mack Publishing Company, Easton, Pennsylvania, 1975. The protective agents can also be given by local administration. Localized administration of methionine protective agents can be carried out by topical application employing pharmaceutical formulations designed for this purpose as is known in the art, local injection, etc.

Additionally, topical administration of the protective agent of the invention includes administration of a cream, gel, paste, solution, patch or other appropriate topical preparation to the skin, for example, by administration of a topical solution or other topical preparation to the outer ear, middle ear or to the round window membrane of the ear, such as by administration of otic drops to the outer ear, middle ear or to the round window membrane of the ear.

Topical administration of the protective agents of the present invention are particularly advantageous for certain types of radiation-induced or chemotherapy-induced tissue damage. In particular, a topical preparation can be applied to the skin to reduce skin damage (e.g., erythema, sunburn, dry desquamation, wet desquamation, swelling, etc.) resulting from radiation exposure. Further, a topical preparation is preferred for treatment of aural mucositis. Particularly, a topical preparation used to treat aural mucositis is applied to the middle ear, outer ear or round window membrane of the ear. More particularly, otic drops are topically administered to treat aural mucositis; otic drops are applied to the middle ear, the outer ear and the round window membrane of the ear.

Administration of the methionine protective agents of the present invention simultaneously with the administration of a platinum-containing chemotherapeutic agent can be accomplished in several ways. For example, each agent can be formulated individually and administered separately at the same time via any of the routes described herein or which are otherwise conventional in the art. Alternatively, both can be contained together in a single dose formulation that can be administered by a single route. As in the case of the platinum-containing chemotherapeutic agent, the dose of methionine protective agent can be administered in a single day.

### Dosages

The protective agents comprising methionine or a methionine-like moiety described herein can be employed in methods for treating human and animal patients undergoing treatment with anti-cancer effective amounts of platinum-containing chemotherapeutic agents to prevent or reduce ototoxicity, weight loss, gastrointestinal toxicity, mucositis, neurotoxicity, alopecia, and to prolong survival. In addition, the protective agents described herein can be employed in methods for treating human and animal patients exposed to radiation levels capable of causing ototoxic effects such as hearing loss, as well as radiation-induced neural damage, alopecia, mucositis and gastrointestinal-disorders. The present methionine protective agents can also improve survival in patients exposed to radiation.

The methods of the present invention comprise administering to the patient an appropriate effective amount of a protective agent comprising methionine or a methionine-like moiety prior to, simultaneously with, or subsequent to administration of a platinum-containing chemotherapeutic agent, or exposure of the patient to radiation. Combinations of these time periods can also be employed.

When administered parenterally, the effective amount of protective agent can be in the range of from about 1.0 mg/kg body weight to about 600 mg/kg body weight. More preferably, the effective amount of protective agent ranges from about 5 mg/kg body weight to about 500 mg/kg body weight, even more preferably from about 10 mg/kg body weight to about 400 mg/kg body weight.

Alternatively, the effective amount of protective agent can be expressed on a mole:mole basis in relation to the anti-cancer effective amount of platinum-containing chemotherapeutic agent. This effective amount can be in the range of from about 4:1 to about 167:1, more preferably from about 4.25:1 to about 100:1, and most preferably from about 4.68:1 to about 20:1, protective agent:platinum-containing chemotherapeutic agent, on a molar basis. A dosing ratio of about 18.75:1 on a molar basis is a preferred ratio.

If necessary, the amounts and ratios described above can be modified for different platinum-containing chemotherapeutic agents, or for exposure to radiation, by routine optimization, including monitoring of effectiveness and titration for the desired effect, by the methods described herein.

When administered orally, the protective agent should be given in an amount that will result in a blood serum level equivalent to that achieved by the parenterally administered dosages set forth above. Such effective oral dosages can easily be determined by one of ordinary skill in the art via conventional *in vitro* or *in vivo* methods such as those described in Remington's Pharmaceutical Sciences, Fifteenth Edition, Mack Publishing Company, Easton, Pennsylvania, 1975.

When administered topically, the effective amount of protective agent is typically administered as a pharmaceutical formulation such as a topical solution. The topical solution typically comprises from about 10 mg/ml to about 50 mg/ml, preferably from about 20 mg/ml to about 30 mg/ml, and most preferably about 25 mg/ml of protective agent.

### Treatment Regimen

In the various methods of the present invention, the effective amount of protective agent can be administered prior to, contemporaneously with, or subsequent to administration of the effective amount of platinum-containing chemotherapeutic agent, or exposure of the patient to radiation. Combinations of these time periods can also be employed. Generally, prior administration of the effective amount of the protective agent can be conducted broadly within the period ranging from as much as 2 days (i.e., about 48 hours or less) before administration of the platinum-containing chemotherapeutic agent or exposure to radiation. Likewise, subsequent administration of the effective amount of the protective agent can be conducted broadly within the period including as much as 2 days (i.e., including about 48 hours or more) after administration of the platinum-containing chemotherapeutic agent or exposure to radiation.

Preferably, prior administration of the effective amount of the methionine protective agent is within about 24 hours before administration of the platinum-containing chemotherapeutic agent or exposure to radiation; with subsequent administration within about 24 hours after administration of the platinum-containing chemotherapeutic agent, or exposure to radiation. More preferably, prior administration is within about 6 hours before administration of the platinum-containing chemotherapeutic agent or exposure to radiation; and subsequent administration is within about 6 hours after administration of the platinum-containing chemotherapeutic agent or exposure to radiation. Even more preferably, prior administration is within about 4 hours before, and subsequent administration is within about 4 hours after administration of the platinum-containing chemotherapeutic agent or exposure to radiation. Even more preferably, prior administration of the effective amount of methionine protective agent is within about 1 hour before, and subsequent administration is within about 1 hour after, administration of the platinum-containing chemotherapeutic agent or exposure to radiation. Still more preferably, prior administration of the effective amount of methionine protective agent is within about one-half hour before, and subsequent administration is within about one-half hour after, administration of the platinum-containing chemotherapeutic agent or exposure to radiation.

The platinum-containing chemotherapeutic agent can be administered parenterally, for example by slow intravenous infusion, or by local injection, as discussed above. The methionine protective agent can be administered as described above, preferably, orally, parenterally by intravenous injection or slow infusion, intraperitoneally or topically.

In a preferred embodiment of the present invention, when treating or preventing mucositis due to exposure to radiation, the effective amount of the protective agent can be administered prior to, simultaneously with, or subsequently to the radiation exposure. For example, it has been found that administering the protective agent to a patient from about 6 hours before the radiation exposure to about 6 hours after the radiation exposure, preferably from about 4 hours before the radiation exposure to about 4 hours after the radiation exposure, more preferably from about 2 hours before the radiation exposure to about 2 hours after the radiation exposure, and even more preferably from about 1 hour before to about 1 hour after the radiation exposure, can significantly ameliorate or prevent mucositis in a human or animal patient.

Delayed toxic effects due to platinum-containing chemotherapeutic agents and radiation exposures have been observed. The protective effects of the present methionine protective agents can be enhanced by administering them in a supplemental manner during the course of the patient's chemotherapy and/or afterwards as necessary or as desired. Thus, the methods described herein can further comprise semi-daily, daily or weekly administration of a supplemental amount of protective agent (i.e., an amount of protective agent which is in addition to the effective amount of protective agent).

Stated another way, it is often beneficial to administer supplemental doses of the protective agents of the present invention so as to maintain effective blood serum levels of the protective agents. Generally, the administration of supplemental amounts of protective agents should result in the blood serum level of the human or animal patient being maintained within at least about 10%, preferably from about 20% to about 70%, and more preferably within about 40%, of the blood serum level of the patient that results from the administration of the effective amount of protective agent. Typically, such supplemental doses are administered within the time frames and dosages set forth above for the effective amount of protective agents, for example, semi-daily, daily or weekly for a period of from about one to fourteen days after the administration of the effective amount.

As with the effective amount of methionine protective agent described above, the supplemental methionine protective agent can generally be administered by any of a wide variety of means. Typically, the supplemental amount of protective agent is administered in the same manner as the effective amount of protective agent. Preferably, the supplemental amount of protective agent is administered orally; parenterally by intravenous injection or slow infusion; intraperitoneally or topically. When administered parenterally, the supplemental amount of the methionine protective agent is preferably in the range of from about 1.0 mg/kg body weight to about 600 mg/kg body weight, more preferably from about 5 mg/kg body weight to about 500 mg/kg body weight, even more preferably from about 10 mg/kg body weight to about 400 mg/kg body weight.

Alternatively, the supplemental amount of methionine protective agent parenterally administered daily or weekly can be expressed on a mole:mole basis in relation to the anti-cancer effective amount of platinum-containing chemotherapeutic agent. This effective amount can be in the range of from about 4:1 to about 167:1, more preferably from about 4.25:1 to about 100:1, and most preferably from about 4.68:1 to about 20:1, methionine protective agent:platinum-containing chemotherapeutic agent, on a molar basis. A dosing ratio of about 18.75:1 on a molar basis is preferred.

Oral or parenteral doses administered daily can be within the ranges listed above. When administered orally, daily or weekly doses should be designed to achieve serum levels equivalent to those achieved by administration of the various parenteral doses described above.

When administered topically, the supplemental amount of protective agent may be administered in the same way as described above for the effective amount, typically as a pharmaceutical formulation such as a topical solution. Generally, the supplemental topical administration comprises applying a topical solution comprising from about 10 mg/ml to about 50 mg/ml, preferably from about 20 mg/ml to about 30 mg/ml, and most preferably about 25 mg/ml of protective agent.

In view of the results presented herein, the medical or veterinary practitioner, by employing the compounds, compositions, and methods described herein, will be able to maintain any of the foregoing parameters in a mammal, especially a human, at a level of from about 70% to about 80% of the pre-chemotherapy or other treatment or exposure level, more preferably from about 80% to about 90% of the pre-chemotherapy or other treatment or exposure level, most preferably from about 90% to about 100% of the pre-chemotherapy or other treatment or exposure level, as measured by standard tests routinely employed in the art. These compounds and methods can also be used for the treatment of domestic pets, such as cats and dogs.

The teachings presented herein permit the design of therapeutic regimens that can be employed to reduce the undesirable side effects of platinum-containing anti-tumor compounds such as CDDP, increase the dosing of such anti-tumor compounds to obtain a higher cancer cure rate, and perhaps include weaker patients in treatment protocols employing such anti-tumor compounds, from which they are currently excluded because they cannot withstand the toxicities associated therewith. The presently disclosed teachings also permit the design of therapeutic regimens useful in preventing or reducing the undesirable ototoxic side effects of radiation, as well as other radiation-induced side effects such as neural damage, alopecia, gastrointestinal disorders, mucositis and decreased patient survival.

Administration of D-methionine before, during, or after administration of antineoplastic effective amounts of platinum-containing anti-tumor compounds such as CDDP, or during various combinations of these time periods, is particularly useful in view of D-methionine's lack of interference with CDDP anti-tumor action. See, Jones and Basinger, Anticancer Res., 9, pp. 1937-42 (1989); and Melvik et al., Inorganica Chimica Acta, 137, pp. 115-18 (1987).

D-methionine and structurally related compounds as claimed can be used in conjunction with platinum-containing antitumor compounds such as CDDP during chemotherapy, and in conjunction with the use of radiation as described herein. These methionine protective agents can also be used to prevent or reduce the ototoxic effects of noise and radiation, as well as other radiation side effects, as described herein.

### Optimization of Treatment Regimen

In the uses described, various parameters associated with the patient's hearing and vestibular systems can be tested by methods well known in the art to establish pretreatment baseline values. After administration of the methionine protective agent, and over the course of chemotherapy and afterwards, ototoxic effects can be monitored by conventional tests, and the results can be compared to those obtained prior to treatment to determine if any change has occurred. If any impairment is observed, the amount and/or time of administration of the protective agent-administered in conjunction with subsequent doses of the platinum-containing chemotherapeutic agent, or exposure to radiation, can be adjusted so as to reduce or prevent further ototoxic changes without substantially diminishing the antineoplastic effectiveness of the platinum-containing chemotherapeutic agent or radiation. Similar modification of treatment parameters in the case of weight loss, gastrointestinal toxicity due to either the platinum-containing chemotherapeutic agent or radiation, neurotoxicity due to either the platinum-containing chemotherapeutic agent or radiation, alopecia due to either the platinum-containing chemotherapeutic agent or radiation, and overall patient condition/survival due to either the platinum-containing chemotherapeutic agent or radiation can be employed to optimize the protective effects of the protective agent with respect thereto. This can be achieved via appropriate testing and comparison of pre- and post-treatment values, e.g., patient weight and patient physical/medical/physiological condition, etc., with protocol adjustments being made as needed.

### EXAMPLES

### EXAMPLE 1

Uses different from the treatment of mucositis or skin damage are not part of the invention.

### Otoprotective Effect of D-methionine

This experiment demonstrates the effectiveness of D-methionine in preventing a variety of different toxic side effects associated with the use of platinum-containing anti-tumor compounds, exemplified by CDDP (cisplatin), in a mammal.

### Materials and Methods

### Animals

As is well known to those of ordinary skill in the art, the rat is a well-accepted experimental animal useful as a model for studies of CDDP toxicity in humans.

Complete data sets were obtained for five groups of five male Wistar rats (280-421 g). All animals were anesthetized with 1 ml/mg IM of Rompun cocktail (a solution containing 86.21 mg/ml ketamine and 2.76 mg/ml xylazine) prior to all injections and testing. Anesthesia was supplemented as needed with half doses throughout testing. The five groups included: a treated control group which received 16 mg/kg CDDP dissolved in normal sterile saline (1 mg of CDDP/ml normal saline; solution pH 6.3) administered by i.p. infusion with a Harvard Apparatus Infusion Pump, over a 30 minute period, an untreated control group that received an equivalent volume of normal saline (pH 6.5) instead of CDDP, and three experimental groups that received either 75, 150, or 300 mg/kg D-methionine dissolved in 3-5 ml of normal saline (solution pH 6.6) delivered by slow (over 1-2 minutes) i.p. injection 30 minutes prior to the same CDDP infusion as the treated control group. Both CDDP (purchased from Sigma Chemical Co., St. Louis) and D-methionine (purchased from Acros Organics, Pittsburgh, PA) were freshly prepared before each experiment. For the treated control group, a total of 10 animals were needed to obtain 5 animals with complete data sets because 50% of the animals did not survive to the end of the study period. Only 5 animals were needed in the untreated control and in each of the D-methionine pretreated groups because all of the animals in each of those groups survived until the end of the study period.

All of the care and use of the animals was approved by the Southern Illinois University School of Medicine Laboratory Animal_Care and Use Committee, and was under the supervision of the Southern Illinois University School of Medicine Unit for Laboratory Animal Medicine.

### Evoked Potentials

Auditory Brainstem Testing (ABR) was used to assess auditory threshold. Testing occurred just prior to administration of the CDDP or saline (with or without a protective agent) and again 3 days later. All testing was performed with the animal in a double walled IAC booth.

Platinum/iridium needle electrodes were placed at the vertex (non-inverting) to a point directly below the ipsilateral pinna (inverting) with a ground electrode placed in the hind leg.

ABR data collection was obtained with a Biologic Traveler system with an additional custom made high frequency stimulator for 14000 Hz. ABR thresholds were measured in response to 100 microsecond clicks and for tonebursts with 1 ms rise/fall and 0 ms plateau gated by a Blackman envelope and centered at the frequencies of 1, 4, 8, and 14 kHz presented at 10/s. An intensity series was obtained for each animal from 100 to 0 dB peak equivalent SPL (peSPL) for click stimuli and Sound Pressure Level (SPL) for tonebursts in 10 dB decrements. The term peSPL means that the amplitude of the click stimulus from the prestimulus baseline to the first peak is equivalent to the SPL of a pure tone stimulus having the same prestimulus baseline to peak amplitude. Threshold was defined as the lowest intensity capable of eliciting a replicable, visually detectable response.

A total of 512 sweeps constituted each average. The recording epoch was 15 ms following stimulus onset. Responses were analogue filtered with a 30-3000 Hz bandpass.

Rectal temperature was monitored throughout recordings, with animal temperature being maintained by a warming pad.

### Electron microscopy

The animals were sacrificed by decapitation while under general anesthesia and cochleae perfused with fixative through the perilymphatic spaces. The primary fixative was 2.5% glutaraldehyde at 4°C in 0.1M phosphate buffer (pH 7.4). A small hole in the otic capsule was hand drilled beneath the first turn with a three sided, sharpened pick. *In vitro* perfusion was performed intermittently within 5 minutes of sacrifice through the small hole in scala tympani, allowing the fluid to exit through the opened oval window. After perfusion fixation, the round window membrane was removed, and the cochleae were immersed in glutaraldehyde and stored in the refrigerator overnight.

After overnight fixation in glutaraldehyde, the cochleae were rinsed in 0.1 M phosphate buffer and gently perfused with the buffer through the perilymphatic spaces by loosely fitting the tube end of the perfusion syringe over the opening drilled in the scala tympani. Cochleae were then rinsed in buffer 3 times. After rinsing, the cochleae were post-fixed by a perfusion of 1.5% OsO₄ (at 4°C) in phosphate buffer in a fume hood. Fixation was continued by immersion and rotation in the same fixative for 15 minutes. The cochleae were rinsed in the same fashion as after glutaraldehyde fixation.

Under the dissecting microscope, the bony capsule of the cochlea was carefully removed.

The tissue was then serially dehydrated in 2 X 50%, 70%, 85%, 95% and 3 X 100% ethanol. Each specimen was dried using Peldri and placed on a stub for sputter coating with 13 nm platinum. The tissue was viewed through a Hitachi S-500 scanning electron microscope and photographs taken on Polaroid type 55 land Film.

Semi-quantitative analysis per turn for the outer hair cells was performed in the following manner: For each turn of the cochlea, apical, middle, and base, a representative sample was examined. For each sample, 11 inner hair cells served as a guide to count a section of 33 outer hair cells or 11 per row. The number of damaged or missing outer hair cells within each sample was then counted.

### Weight

Each animal's weight was measured in an Ohaus triple beam balance scale before administration of the anesthetic for the pretest and again before the post-test 3 days later.

### Statistical Analysis

ABR data were analyzed using a three factor analysis of variance (ANOVA) with one between subject factor (groups) and two within subject factors (frequency and pre-vs. post-test). Each dependent variable was analyzed independently. Tests subsequent to the ANOVA were carried out in accordance with the Tukey HSD procedure. Weight loss and/or gastrointestinal protection was measured using the same type of statistical analysis as the ABR measures. SEM data were analyzed for each turn using a one way analysis of variance with Post-Hoc Tukey HSD analysis. The criterion for statistical significance for all measures was p≤0.01.

### Results

### Hearing loss

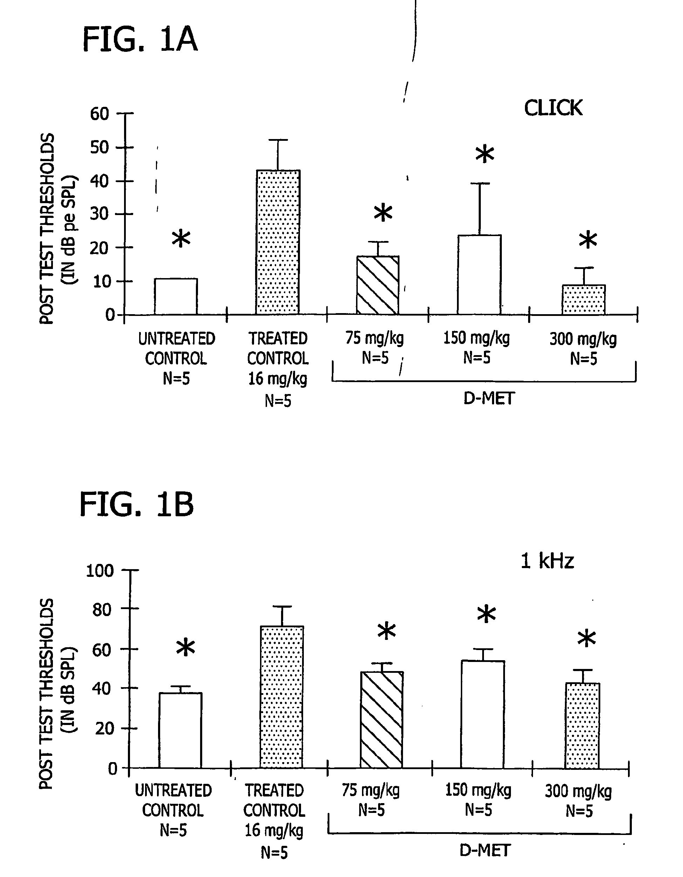

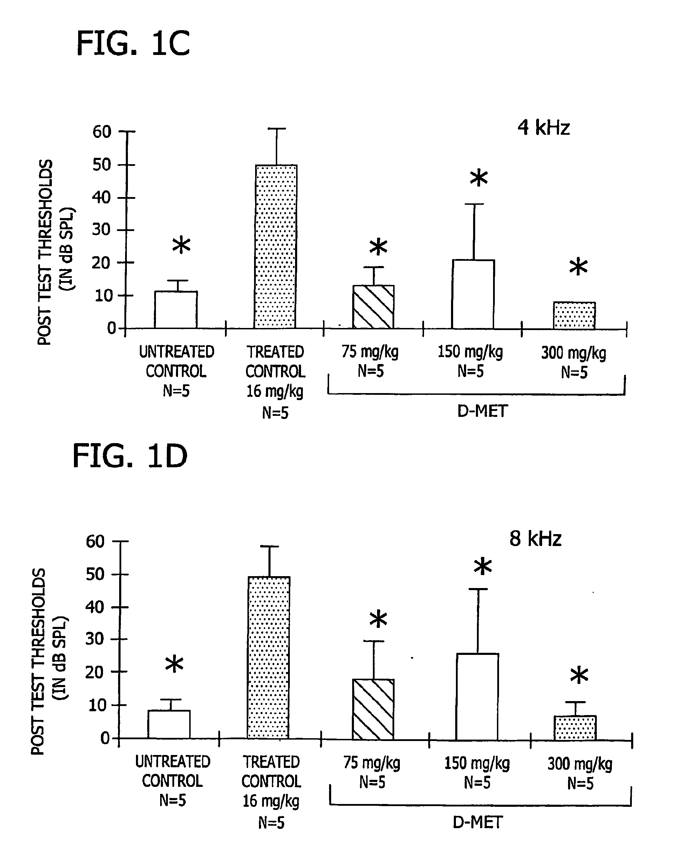

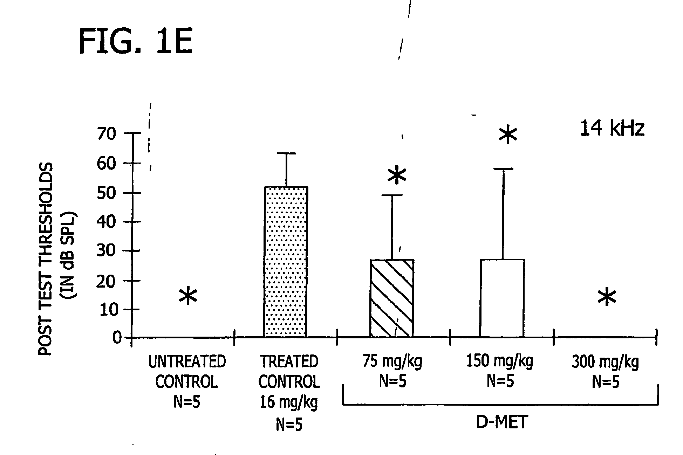

post test ABR hearing thresholds are presented in Figures 1A-1E. As expected, no significant threshold shift in response to any stimulus occurred in the untreated control group, and marked significant threshold shift occurred in response to all stimuli, but particularly for the high frequencies, in the treated control group. For the animals receiving D-methionine prior to the CDDP, 2/5 and 3/5 animals receiving 75 and 150 mg/kg D-methionine, respectively, had complete otoprotection as defined by no significant ABR threshold shift for any stimulus. For the 300 mg/kg D-methionine administration, all 5 animals had complete otoprotection for all stimulus conditions. All experimental groups receiving any level of D-methionine had significantly lower ABR thresholds than the treated control group for all stimuli, as did the untreated control group. This observed protection from hearing loss may occur not only as a result of protection of cochlear mechanisms, but also as a result of protection of the auditory neural pathway (i.e., neuroprotection).

### Histology

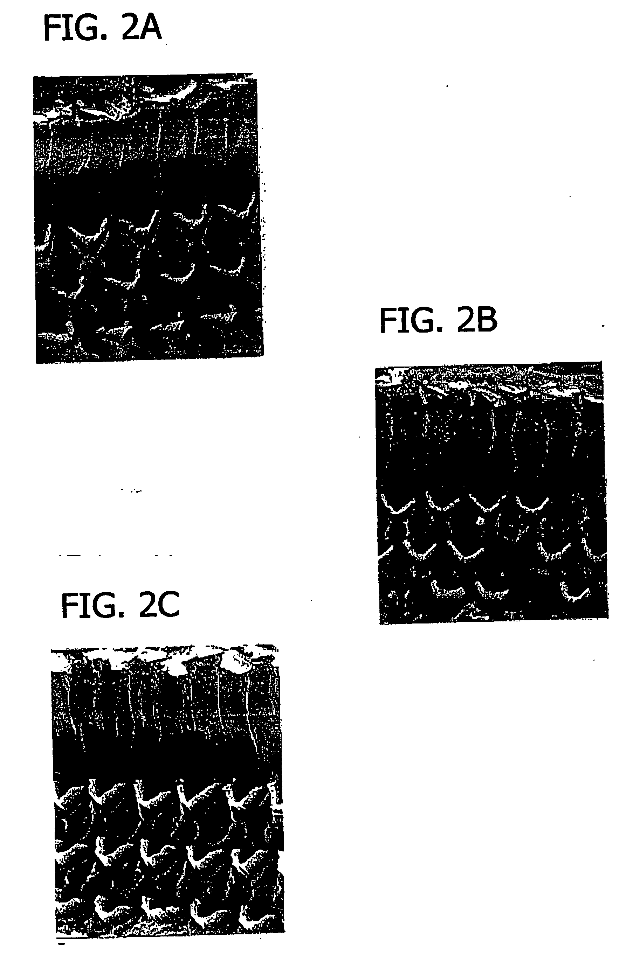

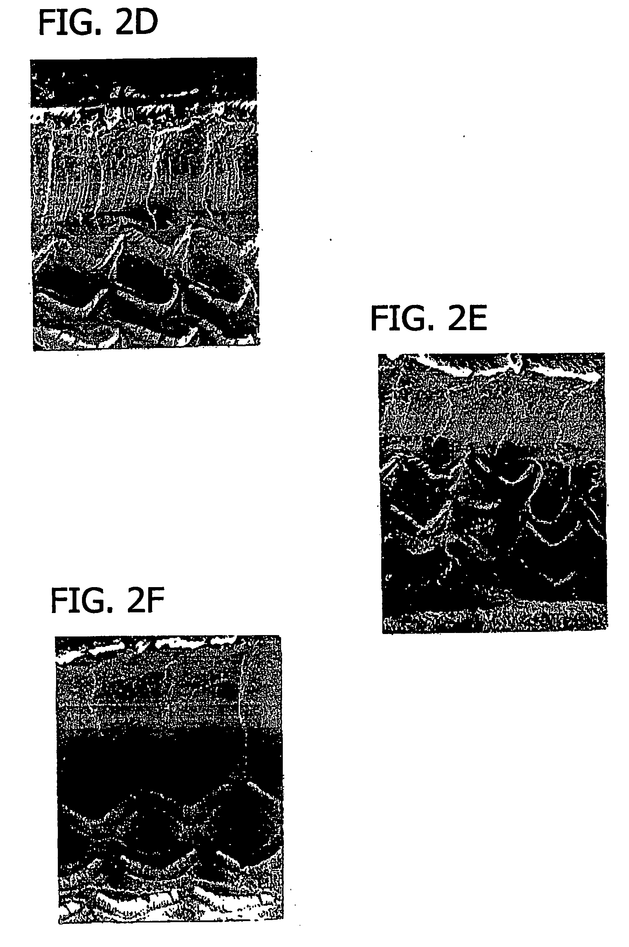

Histologic findings (Figs. 2A-2F) were consistent with the ABR findings. All groups had essentially normal hair cell counts for the apical turn, with no significant difference between groups. For the middle and basal turns, only the treated control group showed significantly different findings from the untreated control group and from the three groups receiving preadministration of D-methionine, with the basal turn being consistently more affected than the middle turn.

### Weight loss

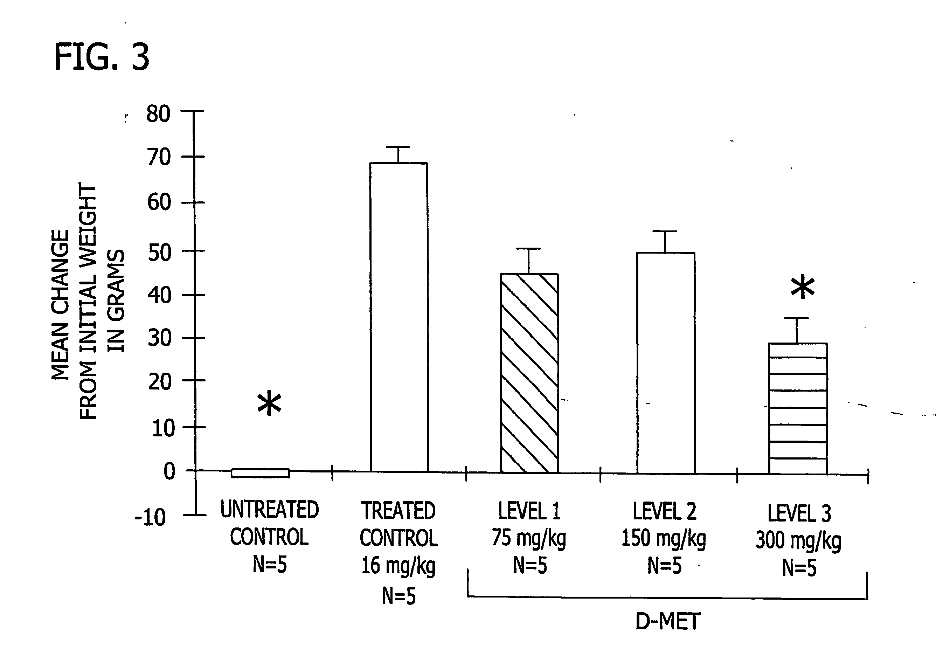

CDDP-induced weight loss diminished as D-methionine dosing increased (Fig. 3). Weight loss in the experimental group receiving 300 mg/kg was significantly less than that in the treated control group. The amount of weight loss across groups was significantly correlated with the amount of threshold shift for all stimuli, with the highest correlation for the 14 kHz stimulus.

### Neuroprotection

Animals receiving D-methionine were noticeably more lively, active, and coordinated on the morning of the third day as compared to the surviving treated control group animals.

### Alopecia

The coats of animals receiving D-methionine were noticeably superior to those of control group animals, and showed significantly less hair loss.

### Survival during the study period

All 15/15 animals receiving any level of D-methionine survived to the end of the study period as compared to 5/10 treated control group animals.

### Discussion

The foregoing results demonstrate that 300 mg/kg D-methionine administered 30 minutes before 16 mg/kg CDDP provides complete otoprotection, as indicated by ABR and histologic findings, while also reducing CDDP-induced weight loss, gastrointestinal toxicity, neurotoxicity, alopecia, and improving survival.

While not intending to be bound to any particular theory, I hypothesize that D-methionine may provide these protective effects by any one or more of a number of different mechanisms.

According to Schweitzer, Laryngoscope, 103, pp. 1-52 (1993), sulfur-containing compounds may prevent CDDP from interacting with intracellular target molecules, the nucleophilic oxygen or sulfur atoms interacting with the electrophilic site of the CDDP, thus displacing or extracting platinum after it is bound. Theoretically, these agents provide protection because of their high affinity for platinum complexes. It is known that CDDP reacts with methionine's sulfhydryl group. See, Lempers et al., Inorgan. Chem., 29, pp. 217-22 (1990).

CDDP may preferentially bind to free D-methionine, thus protecting glutathione. Reduced glutathione is an essential part of the anti-oxidant pathways. CDDP does reduce renal glutathione levels, resulting in increased lipid peroxidation. See, Hannemann et al., Toxicology, 51, pp. 119-32 (1988); Sugihara et al., Jpn. J. Pharm., 44, pp. 71-76 (1987); Sugihara et al., Jpn. J. Pharm., 43, pp. 247-52 (1987); Boogaard et al., Biochem. Pharm., 41(3), pp. 369-75 (1991). CDDP also reduces glutathione levels in the cochlea and inferior colliculus. See, Ravi et al., Pharmacologist, 33(3), p. 217 (1991). More recent work investigated changes specifically in the cochlear antioxidant system. See, Ravi et al., Pharmacol. Toxicol., 76, pp. 386-94 (1995); Rybak et al., Fundam. Appl. Toxicol., 26, pp. 293-300 (1995). Systemic CDDP administration decreased reduced glutathione (GSH) levels, and reduced activity of the enzymes glutathione peroxidase (GSH-Px) and glutathione reductase (GR). Oxidized glutathione or glutathione disulfide (GSSG) was not found, suggesting that the overall glutathione levels decreased rather than merely being oxidized. Ravi et al., Pharmacol. Toxicol., 76, pp. 386-94 (1995) also reported increased cochlear malondialdehyde (MDA) levels, reflecting increased lipid peroxidation. Because CDDP does increase the level of free radicals in general as described by Hannemann et al., Toxicology, 51, pp. 119-32 (1988), preservation of the anti-oxidant system may be critical in preventing CDDP side effects.

D-methionine preadministration may protect the sulfur groups of proteins, including protein bound L-methionine. CDDP binds to the methionine groups in protein and to glutathione. See, Lempers et al., Inorgan. Chem., 29, pp. 217-22 (1990). Schweitzer, Laryngoscope, 103, pp. 1-52 (1993) suggests that platinum binding to protein sulfhydryl groups may cause CDDP nephrotoxicity, accounting for the nephroprotective action of thiols. It is logical that free D-methionine may preferentially bind to CDDP because of the steric hindrance of the protein bound sulfur groups. This protection could occur by preferential binding of the CDDP to D-methionine, or perhaps D-methionine could reverse the Pt binding to the protein-bound methionine and glutathione, as do other sulfur-containing compounds. See, Lempers et al., Inorgan. Chem., 29, pp. 217-22 (1990). Methionine can displace plasma-bound Platinum. See, Alden et al., Chem. Biol. Interact., 48(1), pp. 121-4 (1984).

D-methionine binding to CDDP may also protect free L-methionine (L-Met), an essential amino acid. Parenteral administration of D,L-methionine in humans results in higher plasma levels of the D-isomer. See, Printen et al., Am. J. Clin. Nutr., 32, pp. 1200-05 (1979). Because the D-methionine is less well metabolized than L-Met in humans, it may remain more available for CDDP binding, thus protecting the L-Met for needed protein synthesis, cell activation, and metabolism.

Fortunately, D-methionine does not inhibit CDDP anti-tumor action as determined against the Walker 256 carcinosarcoma in the rat. See, Jones and Basinger, Anticancer Res., 9, pp. 1937-42 (1989). Preadministration of methionine, presumably a racemic mixture, actually sensitized NHIK 3025 in vitro human uterine cervix carcinoma *in situ* cancer cells to CDDP cytotoxicity. See, Melvik et al., Inorganica Chimica Acta, 137, pp. 115-18 (1987).

Several factors may account for D-methionine's CDDP-protective action in nontumor cells as compared to tumor cells. Methionine metabolism is clearly different in tumor and nontumor cells, but how these differences may result in differential CDDP action has not been elucidated. The toxic effects of CDDP may also be different in tumor and nontumor cells. The CDDP anti-tumor effect results primarily from cisplatin's reaction with DNA, primarily at the N-7 bisguanine position. Initially, mono-adducts are formed, followed by rapid intra-strand cross-linking, causing cytotoxicity. See, Tognella, Cancer Treat. Rev., 17, pp. 139-42 (1990). The binding of platinum to cytosolic ligands and nucleoprotein fractions may also play a role, but the receptors and interactions are not yet defined. See, Schweitzer, Laryngoscope, 103, pp. 1-52 (1993). Significant DNA binding in normal cells is less likely because fewer DNA replication forks are open at any point in time, unlike in rapidly dividing tumor cells. In nontumor cells, the toxic effects may be largely secondary to the binding with amino acids, either free or protein-bound, and deactivation of the antioxidant pathway, as described above.

The timing of CDDP reactions may also be different in tumor and nontumor cells. CDDP uptake by the Walker 256 carcinosarcoma in the rat is very rapid, occurring in the first few minutes after administration, followed by a rapid redistribution that is complete within 15 minutes after injection. See, Jones and Basinger, Anticancer Res., 9, pp. 1937-42 (1989). Because the uptake of CDDP into tumor cells is very rapid, the binding to the DNA bisguanine groups, particularly at the open replication forks, may occur more rapidly than the reaction of CDDP with methionine.

Although CDDP uptake into the kidney is also rapid (see, Jones and Basinger, Anticancer Res., 9, pp. 1937-42 (1989)), CDDP binding to protein is relatively slow. As reviewed by Schweitzer, supra, following IV cisplatin administration, 90% of cisplatin is protein-bound within 2 hours, with half-lives of 25 to 50 minutes and 53 to 73 hours for unbound and bound platinum, respectively. Platinum tissue levels decline slowly. Platinum may still be measured over a week after high dosage administration, and bound fragments may still be present when the patient starts the next treatment cycle. Platinum uptake in the stria vascularis and the organ of Corti increases at least over a 24 hour period, which may underlie the dose-related cumulative ototoxicity, but may also allow time for CDDP binding to D-methionine before uptake into the cochlea.

However, the CDDP toxicities both in tumor and nontumor cells are complex, and many factors may be involved in D-methionine's protective action.

A positive correlation between weight loss and outer hair cell loss in guinea pigs has been demonstrated by Tange et al. and Hoeve et al., but both studies noted marked intersubject variability. See, Tange et al., "The Cortitoxic Effect of Cis-Platinum in the Guinea Pig," Arch. Oto-Rhino-Laryngol. 237, pp. 17-26 (1982); and Hoeve et al., "Correlations between Cis-Platinum Dosage and Toxicity in a Guinea Pig Model," Arch. Otorhinolaryngol., 245, pp. 98-102 (1988). The data presented above reveal a positive correlation between weight loss and threshold loss that increased as stimulus frequency increased. The significant reduction in weight loss with 300 mg/kg D-methionine preadministration suggests that D-methionine also alleviates some of the gastrointestinal toxicities of CDDP. The amelioration in weight loss by D-methionine could also be related to a decrease in nephrotoxicity or other factors.

The elimination of CDDP mortality in this study by preadminstration of any of the three D-methionine levels demonstrates a marked improvement in the overall health status of the animals. D-methionine preadministration may therefore be useful in shifting the LD₅₀ level of CDDP and other platinum-containing anti-tumor agents, permitting the safe use of higher levels of these agents during chemotherapy, with potential improvement of the cancer cure rate.

### EXAMPLE 2

This example demonstrates the use of D-methionine for protecting cells during radiation cancer therapy. The experiment was conducted by investigating the sensitivity of a human salivary gland cell line to radiation sensitivity in the presence and absence of D-methionine. Seven conditions were used comprising an untreated control, a control treated with D-methionine (1 mg/ml) alone, a control treated with ionizing radiation (10 Gy) alone, and four sets which were treated with D-methionine six hours before being irradiated with ionizing radiation (10 Gy). The four conditions treated with D-methionine before radiation exposure were treated with 1 mg/ml D-methionine, 0.5 mg/ml D-methionine, 0.2 mg/ml D-methionine and 0.1 mg/ml D-methionine respectively.

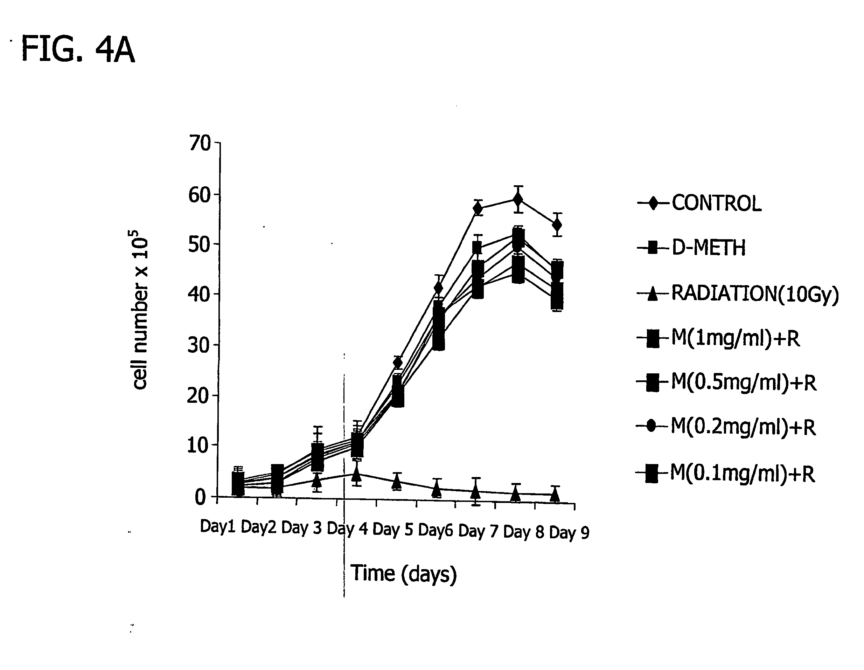

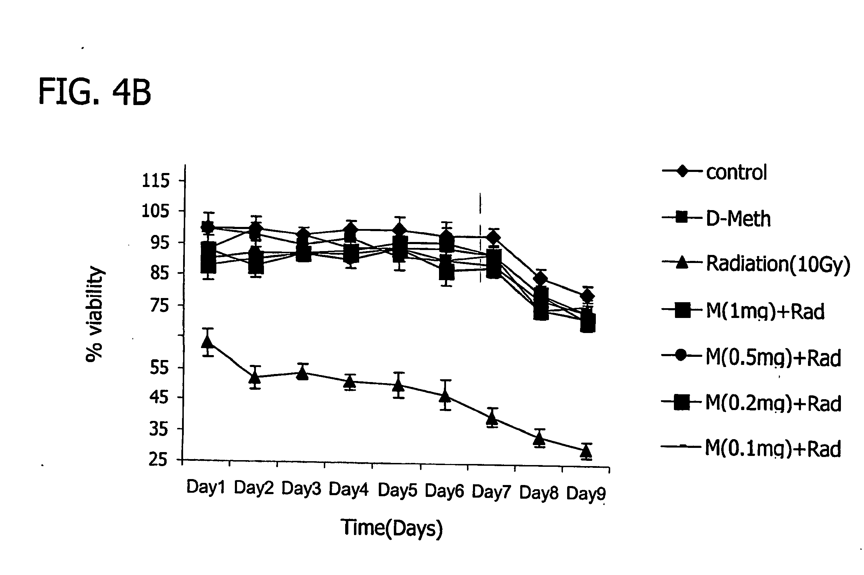

The human salivary gland derived cells were inoculated into 10 cm² dishes and monitored for growth rates and viability by counting over 9 days. Each set of conditions included 9 dishes of cells, one for each day. Each day, a representative dish was harvested and the number of cells in the dish were counted by trypan blue exclusion. Results for cell growth rates and viability of irradiated and control cells in the presence or absence of D-methionine are shown in Figs. 4A and 4B.

Referring to the Fig. 4A, the untreated control cells grew logarithmically for 7 days after which they become stationary. In contrast, the control cells irradiated with 10 Gy failed to undergo log phase growth. When viability was examined in these cultures (Fig. 4B), untreated control cells had 80% to 95% viability throughout the 7 days, while the irradiated control cells had only 65% viability at day one and viability decreased to 25% on day 7. When irradiated cells were pre-treated with 1.0, 0.5, 0.2 and 0.1 mg/ml D-methionine, the cells underwent log phase growth despite having being irradiated with 10 Gy (Figure 4A). The treated cells also maintained a high viability (85-95%, Fig. 4B). These results demonstrate that D-methionine protects cells from the cytotoxic effects of ionizing irradiation.

### EXAMPLE 3

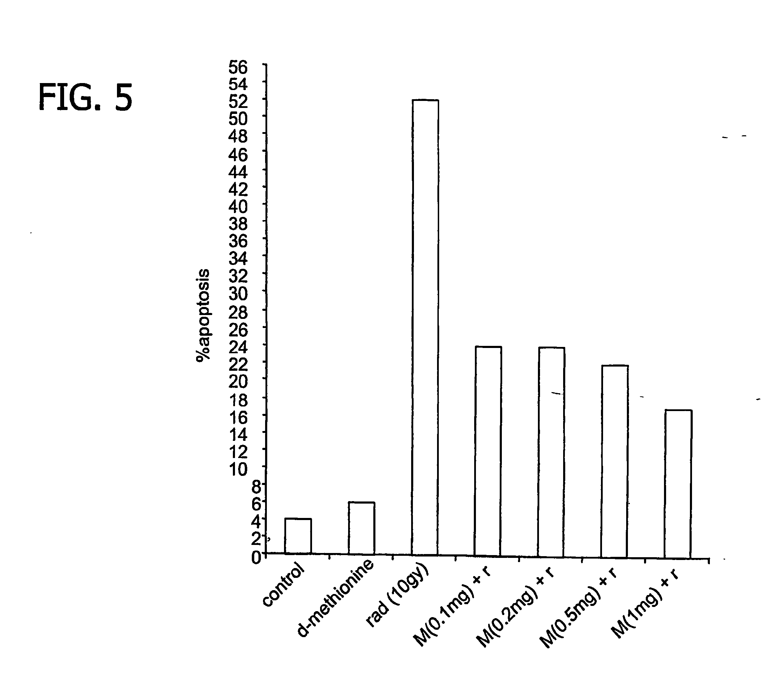

This example demonstrates the use of D-methionine for protecting cells from apoptosis during radiation cancer therapy. Human salivary gland epithelial cells were plated and stained with propidium iodide to determine the rate of apoptosis after 24 hours. One set was untreated as a control and another was irradiated with 10 Gy the following morning. A third set was pre-treated with various doses of D-methionine (1.0 mg/ml, 0.5 mg/ml, 0.2 mg/ml and 0.1 mg/ml) six hours prior to radiation treatment (10 Gy). Untreated cells which were irradiated demonstrated the presence of condensed, pyknotic nuclei which is a hallmark of apoptosis when cells are irradiated. In contrast, pre-treatment of cells with D-methionine prior to irradiation was shown to block apoptosis as few cells were seen to contain pyknotic, condensed nuclei. Multiple fields were used to determine the percentage of cells having an apoptotic phenotype, from which the calculated percentages were graphed as shown in Fig. 5. Briefly, irradiation of HSG cells resulted in approximately 60% apoptosis after 24 Hrs, while pretreatment with various concentrations of D-methionine showed significant decreases in apoptosis to about 20%.

### EXAMPLE 4

This example demonstrates the use of D-methionine for the treatment or prevention of radiation-induced oral mucositis. The experiment was conducted using a mouse model of radiation-induced lip erythema. Four groups of mice (n=5) were used. The first group (Group A) was an untreated, control group. The second group (Group B) was irradiated with ionizing radiation (6 Gy/day) for 5 days. The third group (Group C) was irradiated with ionizing radiation (6 Gy/day) for five days and was treated with D-methionine (150 mg/kg) six hours prior to irradiation on each day. The fourth group (Group D) was irradiated with ionizing radiation (6 Gy/day) for five days and was treated with D-methionine (150 mg/kg) one hour after irradiation on each day.

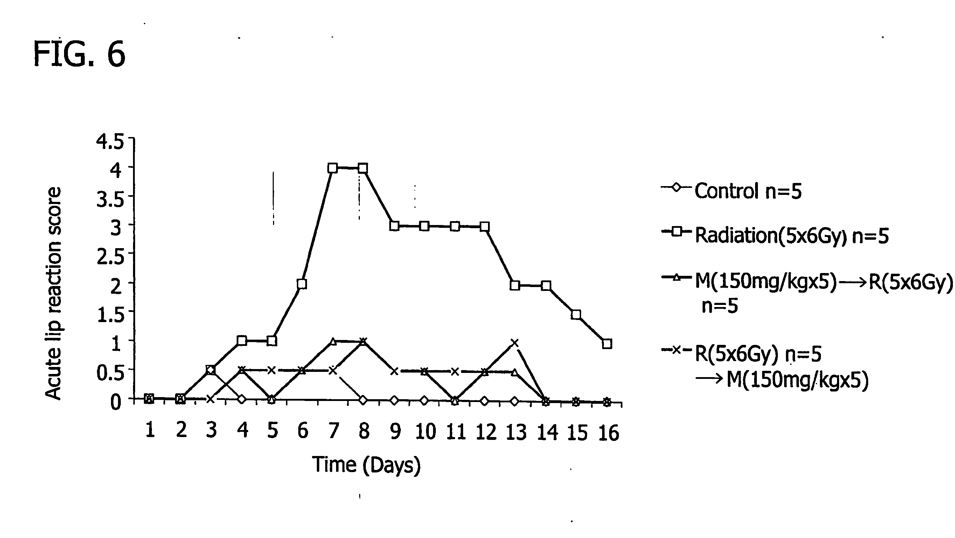

Two independent observers were used to score the experiment and express the results quantitatively as shown in Fig. 6. The irradiation of mice,resulted in lip erythema (i.e., reddening, swelling, desquamation of the lips). Both pre-treatment and post-treatment of the animals with D-methionine prevented the occurrence of lip erythema.

The experiment further determined that post-irradiation administration of D-methionine does not interfere with antitumor activity of radiation therapy. In addition, either pre or post treatment of tumor bearing animals with D-methionine (150 mg/Kg x 5, i.p.) did not interfere with antitumor activity of ionizing radiation.

The results of the experiment clearly demonstrate that D-methionine protects mice from radiation-induced lip erythema (a model for oral mucositis). In addition, the administration of D-methionine before and/or after radiation therapy is demonstrated to be effective without interfering with antitumor activity of ionizing radiation. Without being held to a particular theory, it is believed that D-methionine may selectively protect normal host cell mitochondrial membranes from radiation damage, thereby protecting the cells from apoptosis. However, the data suggests that D-methionine does not protect the mitochondrial damage resulting in cell death in tumor cells. These animal data provide a good rationale for the evaluation of D-methionine for the prevention and treatment of oral mucositis induced by radiation treatment.

## Claims

1. Use of a protective agent comprising methionine or a methionine-like moiety to prepare a pharmaceutical composition for the prevention or reduction of mucositis in a human or animal patient wherein the human or animal patient is exposed to radiation or is undergoing treatment with a chemotherapeutic effective amount of an antineoplastic agent and wherein the methionine-like moiety has the structural formula: 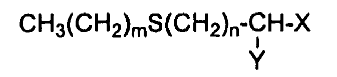 wherein m is an integer from 0 to 3; n is an integer from 1 to 3; X = -OR¹, -OCOR¹, -COOR¹, -CHO, -CH(OR¹)₂, or -CH₂OH; Y = -NR²R³; R¹ = H or a substituted or unsubstituted, straight or branched chain alkyl group having 1 to 6 carbon atoms; R² = H or a substituted or unsubstituted, straight or branched chain acyl group having 1 to 6 carbon atoms; and R³ = H or a substituted or unsubstituted, straight or branched chain acyl group having 1 to 6 carbon atoms; or
a pharmaceutically acceptable salt thereof.

2. Use of a protective agent comprising methionine or a methionine-like moiety to prepare a pharmaceutical composition for the prevention or reduction of skin damage in a human or animal patient wherein said human or animal patient is overexposed to radiation and wherein the methionine-like moiety has the structural formula: 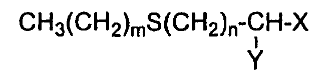 wherein m is an integer from 0 to 3; n is an integer from 1 to 3; X = -OR¹, -OCOR¹, -COOR¹, -CHO, -CH(OR¹)₂, or -CH₂OH; Y = -NR²R³; R¹ = H or a substituted or unsubstituted, straight or branched chain alkyl group having 1 to 6 carbon atoms; R² = H or a substituted or unsubstituted, straight or branched chain acyl group having 1 to 6 carbon atoms; and R³ = H or a substituted or unsubstituted, straight or branched chain acyl group having 1 to 6 carbon atoms; or a pharmaceutically acceptable salt thereof;
wherein the protective agent is administered orally to said patient.

3. A use as set forth in claim 1 wherein the antineoplastic agent is an anti-tumor platinum-coordination compound.

4. A use as set forth in claim 2 wherein said human or animal patient is undergoing treatment with a chemotherapeutic effective amount of an anti-tumor platinum-coordination compound.

5. A use as set forth in claim 1 wherein said antineoplastic agent is L-asparaginase, Ara-C, busulfan, cyclophosphamide, docetaxel, doxorubicin, edatrexate, etoposide, fludarabine, fluorouracil, gencitabine, idarubicin, ifosamide, irinotecan, leucovorin, melphalan, methotrexate, mitomycin C, mitoxantrone, oxaliplatin, paclitaxel, raltitrexed, thiotepa, or vinorelbine.

6. A use as set forth in claim 2, wherein the protective agent is selected from the group consisting of L-methionine, a mixture of D-methionine and L-methionine, normethionine, homomethionine, methioninol, ethionine, a pharmaceutically acceptable salt thereof, and a combination thereof.

7. A use as set forth in claim 1, wherein the protective agent is selected from the group consisting of L-methionine, a mixture of D-methionine and L-methionine, normethionine, homomethionine, methioninol, ethionine, a pharmaceutically acceptable salt thereof, and a combination thereof.

8. A use as set forth in claim 7, wherein the protective agent is D-methionine.

9. A use as set forth in claim 7, wherein the protective agent is L-methionine.

10. A use as set forth in claim 7, wherein the protective agent is D,L-methionine.

11. A use as set forth in claim 1 or 4 wherein the anti-tumor platinum-coordination compound or antineoplastic agent is *cis-*diamminedichloro-platinum(II), *trans*-diaminedichloro-platinum(II), *cis*-diamine-diaquaplatinum(II)-ion, *cis*-diaminedichloroplatinum(II)-ion, chloro(diethylenetriamine)-platinum(II) chloride, dichloro(ethylenediamine)-platinum(II), diammine(1,1-cyclobutanedicarboxylato)-platinum(II) (carboplatin), spiroplatin, dichlorotrans-dihydroxybisisopropolamine platinum IV (iproplatin), diammine(2-ethylmalonato)-platinum(II), ethylenediamine-malonatoplatinum(II), aqua(1,2-diaminodiclohexane)-sulfatoplatinum(II), (1,2-diaminocyclohexane)malonato-platinum(II), (4-carboxyphthalato)(1,2-diaminocyclo-hexane)-platinum(II), (1,2-diaminocyclohexane)-(isocitrato)platinum(II), (1,2-diaminocyclohexane)-*cis*(pyruvato)platinum(II), or (1,2-diaminocyclohexane)-oxalatoplatinum(II).

12. A use as set forth in claim 3, wherein the protective agent is to be administered prior to, simultaneously with, or subsequently to administration of said chemotherapeutic effective amount of an anti-tumor platinum-coordination compound.

13. A use as set forth in claim 1, wherein the protective agent is to be administered prior to, simultaneously with, or subsequently to said radiation exposure.

14. A use as set forth in claim 1, wherein the protective agent is to be administered prior to, simultaneously with, or subsequently to administration of said chemotherapeutic effective amount of an antineoplastic agent.

15. A use as set forth in claim 3 or 4, wherein a supplemental amount of the protective agent is to be administered after the administration of said effective amount of said protective agent.

16. A use as set forth in claim 1 for preventing or reducing oral mucositis.

17. A use as set forth in claim 2 wherein said skin damage comprises erythema.

18. A use as set forth in claim 2 wherein said skin damage comprises sunburn.

19. A use as set forth in claim 2 wherein said skin damage comprises dry desquamation, wet desquamation and swelling.

## Patentansprüche

1. Verwendung eines Schutzmittels mit Methionin oder einem Methionin-artigen Teil zum Herstellen einer pharmazeutischen Zusammensetzung für die Verhütung oder Reduzierung von Mukositis in einem menschlichen oder tierischen Patienten, wobei der menschliche oder tierische Patient Strahlung ausgesetzt wird oder eine Behandlung mit einer chemotherapeutischen wirksamen Menge eines antineoplastischen Mittels erfährt und wobei der Methionin-artige Teil die Strukturformel 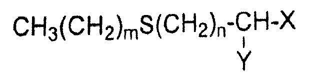 hat, worin m eine ganze Zahl von 0 bis 3 ist, n eine ganze Zahl von 1 bis 3 ist, X = -OR¹, -OCOR¹, -COOR¹, -CHO, -CH(OR¹)₂ oder -CH₂OH ist, Y = -NR²R³; R¹= H oder eine substituierte oder unsubstituierte, gerad- oder verzweigtkettige Alkylgruppe mit 1 bis 6 Kohlenstoffatomen, R² = H oder eine substituierte oder unsubstituierte, gerad- oder verzweigtkettige Acylgruppe mit 1 bis 6 Kohlenstoffatomen, und R³ = H oder eine substituierte oder unsubstituierte, gerad- oder verzweigtkettige Acylgruppe mit 1 bis 6 Kohlenstoffatomen, oder
deren pharmazeutisch zulässiges Salz.

2. Verwendung eines Schutzmittels mit Methionin oder einem Methionin-artigen Teil zum Herstellen einer pharmazeutischen Zusammensetzung für die Verhütung oder Reduzierung von Hautschädigung in einem menschlichen oder tierischen Patienten, wobei der menschliche oder tierische Patient Strahlung überausgesetzt wird und wobei der Methionin-artige Teil die Strukturformel 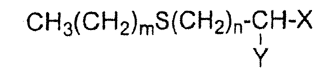 hat, worin m eine ganze Zahl von 0 bis 3 ist, n eine ganze Zahl von 1 bis 3 ist, X = -OR¹, -OCOR¹, -COOR¹, -CHO, -CH(OR¹)₂ oder -CH₂OH, Y = -NR²R³, R¹ = H oder eine substituierte oder unsubstituierte, gerad- oder verzweigtkettige Alkylgruppe mit 1 bis 6 Kohlenstoffatomen, R² = H oder eine substituierte oder unsubstituierte, gerad- oder verzweigtkettige Acylgruppe mit 1 bis 6 Kohlenstoffatomen, R³ = H oder eine substituierte oder unsubstituierte, gerad- oder verzweigtkettige Acylgruppe mit 1 bis 6 Kohlenstoffatomen, oder deren pharmazeutisch zulässiges Salz,
wobei das Schutzmittel dem Patienten oral verabreicht wird.

3. Verwendung nach Anspruch 1, bei der das antineoplastische Mittel eine Antitumor-Platinkoordinationsverbindung ist.

4. Verwendung nach Anspruch 2, bei der der menschliche oder tierische Patient eine Behandlung mit einer chemotherapeutischen wirksamen Menge einer Antitumor-Platinkoordinationsverbindung erfährt.

5. Verwendung nach Anspruch 1, bei der das antineoplastische Mittel L-Asparaginase, Ara-C, Busulfan, Cyclophosphamid, Docetaxel, Doxorubicin, Edatrexat, Etoposide, Fludarabin, Fluorouracil, Gencitabin, Idarubicin, Ifosamid, Irinotecan, Leucovorin, Melphalan, Methotrexat, Mitomycin C, Mitoxantron, Oxaliplatin, Paclitaxel, Raltitrexed, Thiotepa oder Vinorelbin ist.

6. Verwendung nach Anspruch 2, bei der das Schutzmittel aus der Gruppe ausgewählt ist, die aus L-Methionin, einem Gemisch von D-Methionin und L-Methionin, Normethionin, Homomethionin, Methioninol, Ethionin, einem pharmazeutisch zulässigen Salz daraus und einer Kombination daraus besteht.

7. Verwendung nach Anspruch 1, bei der das Schutzmittel aus der Gruppe ausgewählt ist, die aus L-Methionin, einem Gemisch von D-Methionin und L-Methionin, Normethionin, Homomethionin, Methioninol, Ethionin, einem pharmazeutisch zulässigen Salz daraus und einer Kombination daraus besteht.

8. Verwendung nach Anspruch 7, bei der das Schutzmittel D-Methionin ist.

9. Verwendung nach Anspruch 7, bei der das Schutzmittel L-Methionin ist.

10. Verwendung nach Anspruch 7, bei der das Schutzmittel D,L-Methionin ist.

11. Verwendung nach Anspruch 1 oder 4, bei der die Antitumor-Platinkoordinationsverbindung oder das antineoplastische Mittel cis-Diammindichlor-platin(II), trans-Diamindichlor-platin(II), cis-Diamin-diaquaplatin(II)-Ion, cis-Diamindichlorplatin(II)-Ion, Chlor(diethylentriamin)-platin(II)-chlorid, Dichlor(ethylendiamin)-platin(II), Diammin(1,1-cyclobutandicarboxylato)-platin(II) (Carboplatin), Spiroplatin, Dichlortrans-dihydroxybisisopropolaminplatin IV (Iproplatin), Diammin(2-ethylmalonato)-platin(II), Ethylendiaminmalonatoplatin(II), Aqua(1,2-diaminodiclohexan)sulfato-platin(II), (1,2-Diaminocyclohexan)malonato-platin(II), (4-Carboxy-phthalato)(1,2-diaminocyclo-hexan)-platin(II), (1,2-Diaminocyclohexan)-(isocitrato)platin(II), (1,2-Diaminocyclohexan)-cis(pyruvato)-platin(II) oder (1,2-Diaminocyclohexan)-oxalatoplatin(II) ist.

12. Verwendung nach Anspruch 3, bei der das Schutzmittel vor, gleichzeitig mit oder nach Verabreichung der chemotherapeutischen wirksamen Menge einer Antitumor-Platinkoordinationsverbindung zu verabreichen ist.

13. Verwendung nach Anspruch 1, bei der das Schutzmittel vor, gleichzeitig mit oder nach der Strahlungsaussetzung zu verabreichen ist.

14. Verwendung nach Anspruch 1, bei der das Schutzmittel vor, gleichzeitig mit oder nach Verabreichung der chemotherapeutischen wirksamen Menge eines antineoplastischen Mittels zu verabreichen ist.

15. Verwendung nach Anspruch 3 oder 4, bei der eine zusätzliche Menge des Schutzmittels nach der Verabreichung der wirksamen Menge des Schutzmittels zu verabreichen ist.

16. Verwendung nach Anspruch 1 zur Verhütung oder Reduzierung oraler Mukositis.

17. Verwendung nach Anspruch 2, bei der die Hautschädigung Erythem umfaßt.

18. Verwendung nach Anspruch 2, bei der die Hautschädigung Sonnenbrand umfaßt.

19. Verwendung nach Anspruch 2, bei der die Hautschädigung trockene Abschuppung, feuchte Abschuppung und Schwellung umfaßt.

## Revendications

1. Utilisation d'un agent protecteur comprenant un groupe méthionine ou de type méthionine pour préparer une composition pharmaceutique destinée à la prévention ou la réduction de la mucosite chez une patient humain ou animal, dans laquelle le patient humain ou animal est exposé un rayonnement ou subit un traitement avec une quantité chimiothérapeutique efficace d'un agent antinéoplasique et dans laquelle le groupe de type méthionine répond à la formule structurelle : 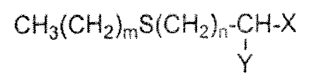 dans laquelle m est un entier de 0 à 3 ; n est un entier de 1 à 3 ; X = -OR¹, - OCOR¹, -COOR¹, -CHO, -CH(OR¹)₂ ou -CH₂OH , Y = -NR²R² ; R¹ = H ou un groupe alkyle à chaîne droite ou ramifiée, substitué ou non substitué, comportant 1 à 6 atomes de carbone; R² = H ou un groupe acyle à chaîne droite ou ramifiée, substitué ou non substitué, comportant 1 à 6 atomes de carbone : et R³ = H ou un groupe acyle à chaîne droite ou ramifiée, substitué ou non substitué, comportant 1 à 6 atomes de carbone : ou
d'un sel pharmaceutiquement acceptable de celui-ci.

2. Utilisation d'un agent protecteur comprenant un groupe méthionine ou de type méthionine pour préparer une composition pharmaceutique destinée à la prévention ou la réduction d'un dommage cutané chez un patient humain ou animal, dans laquelle ledit patient humain ou animal est surexposé à un rayonnement et dans laquelle le groupe de type méthionine répond à la formule structurelle : 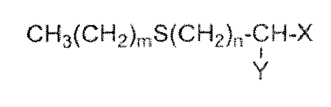 dans laquelle m est un entier de 0 à 3 : n est un entier de 1 à 3 ; X = -OR¹, - OCOR¹, -COOR¹, -CHO, -CH(OR¹)₂ ou -CH₂OH ; Y = -NR²R³; R¹ = H ou un groupe alkyle à chaîne droite ou ramifiée, substitué ou non substitué, comportant 1 à 6 atomes de carbone ; R² = H ou un groupe acyle à chaîne droite ou ramifiée, substitué ou non substitué, comportant 1 à 6 atomes de carbone : et R³ = H ou un groupe acyle à chaîne droite ou ramifiée, substitué ou non substitué, comportant 1 à 6 atomes de carbone ; ou d'un sel pharmaceutiquement acceptable de celui-ci ;
dans laquelle l'agent protecteur est administré par voie orale audit patient.

3. Utilisation selon la revendication 1, dans laquelle l'agent antinéoplasique est un composé de coordination du platine antitumoral.

4. Utilisation selon la revendication 2, dans laquelle ledit patient humain ou animal subit un traitement avec une quantité chimiothérapeutique efficace d'un composé de coordination du platine antitumoral.

5. Utilisation selon la revendication 1, dans laquelle ledit agent antinéoplasique est la L-asparaginase, l'Ara-C, le busulfan, le cyclophosphamide, le docétaxel, la doxorubicine, l'édatrexate, l'étoposide, la fludarabine, le fluorouracile, la gencitabine, l'idarubicine, l'ifosamide, l'irinotécan, la leucovorine, le melphalan, le méthotrexate, la mitomycine C, la mitoxantrone, l'oxaliplatine, le paclitaxel, le raltitrexed, le thiotepa ou la vinorelbine.

6. Utilisation selon la revendication 2, dans laquelle l'agent protecteur est choisi dans le groupe constitué par la L-méthionine, un mélange de D-méthionine et de L-méthionine, la norméthionine, l'homométhionine, le méthioninol, l'éthionine, un sel pharmaceutiquement acceptable de ceux-ci, et une combinaison de ceux-ci.

7. Utilisation selon la revendication 1, dans laquelle l'agent protecteur est choisi dans le groupe constitué par la L-méthionine, un mélange de D-méthionine et de L-méthionine, la norméthionine, l'homométhionine, le méthioninol, l'éthionine, un sel pharmaceutiquement acceptable de ceux-ci, et une combinaison de ceux-ci.

8. Utilisation selon la revendication 7, dans laquelle l'agent protecteur est la D-méthionine.

9. Utilisation selon la revendication 7, dans laquelle l'agent protecteur est la L-méthionine.

10. Utilisation selon la revendication 7, dans laquelle l'agent protecteur est la D,L-méthionine.

11. Utilisation selon la revendication 1 ou 4, dans laquelle le composé de coordination du platine antitumoral ou l'agent antinéoplasique est le *cis-*diaminedichloro-platine (II), le *trans*-diaminedichloro-platine (II), l'ion *cis*-diamine-diaquaplatine (II), l'ion *cis*-diamine-dichloroplatine (II), le chlorure de chloro-(diéthylènetriamine)-platine (II), le dichloro(éthylènediamine)-platine (II), le diamine(1,1-cyclobutanedicarboxylato)-platine (II) (carboplatine), le spiroplatine, le dichlorottrans-dihydroxybisisopropolamine platine IV (iproplatine), le diamine(2-éthylmalonato)-platine (II), l'éthylènediamine-malonatoplatine (II), l'aqua(1,2-diaminodicyclohexane)-sulfatoplatine (II), le (1,2-diaminocyclohexane)malonatoplatine (II), le (4-carboxyphtalato)(1,2-diaminocyclo-hexane)-platine (II), le (1,2-diaminocyclohexane)-(isocitrato)platine (II), le (1,2-diaminocyclohexane)-*cis*(pyruvato)platine (II) ou le (1,2-diaminocyclohexane)-oxalatoplatine (II).

12. Utilisation selon la revendication 3, dans laquelle l'agent protecteur doit être administré avant, pendant ou après l'administration de ladite quantité chimiothérapeutique efficace d'un composé de coordination du platine antitumoral.

13. Utilisation selon la revendication 1, dans laquelle l'agent protecteur doit être administré avant, pendant ou après l'exposition à un rayonnement.

14. Utilisation selon la revendication 1, dans laquelle l'agent protecteur doit être administré avant, pendant ou après l'administration de ladite quantité chimiothérapeutique efficace d'un agent antinéoplasique.

15. Utilisation selon la revendication 3 ou 4, dans laquelle une quantité complémentaire de l'agent protecteur doit être administrée après l'administration de ladite quantité efficace dudit agent protecteur.

16. Utilisation selon la revendication 1, destinée à prévenir ou réduire la mucosite orale.

17. Utilisation selon la revendication 2, dans laquelle ledit dommage cutané comprend l'érythème.

18. Utilisation selon la revendication 2, dans laquelle ledit dommage cutané comprend les coups de soleil.

19. Utilisation selon la revendication 2, dans lequel le dommage cutané comprend la desquamation sèche, la desquamation humide et le gonflement.
